# EUROPEAN PATENT APPLICATION

(11) **EP 4 641 574 A1**
(43) Date of publication of application: **29.10.2025**
(21) Application number: 23895080.2
(22) Date of filing: 24.11.2023
(51) Int. Cl.: G16B 40/00, G16B 30/10, G16B 45/00, G16B 5/00

(54) **METHOD FOR ESTIMATING ORGANISM OR HOST, METHOD FOR ACQUIRING MODEL FOR ESTIMATING ORGANISM OR HOST, AND COMPUTER DEVICE FOR PERFORMING SAME**

(30) Priority: 25.11.2022 KR 20220160091
(71) Applicant: Seegene, Inc., Seoul 05548 (KR)
(72) Inventor: JEONG, Ha Neul, Seoul 05548 (KR); JANG, Jong Ha, Seoul 05548 (KR); KIM, Hyun Ho, Seoul 05548 (KR); LEE, Gwang Ho, Seoul 05548 (KR)
(74) Representative: Viering, Jentschura & Partner mbB Patent- und Rechtsanwälte
(86) International application number: PCT/KR2023/019095
(87) International publication number: WO 2024/112153

(57) **Abstract**

According to an embodiment, disclosed is a computer-implemented method performed by a computer device using a memory, a processor, and one or more programs stored in the memory and configured to be executed by the processor, the method comprising: accessing an assumption model obtained by fine-tuning a pre-learned model; providing a nucleic acid sequence to the assumption model; and assuming an organism carrying the nucleic acid sequence or a host of the organism from the assumption model. A representative figure may be FIG. 1.

## Description

### [Technical Field]

The present invention relates to a method for estimating an organism or a host, a method for acquiring a model for estimating an organism or a host, and a computer device for performing same.

### [Background Art]

Molecular diagnostic technologies are currently growing rapidly in the in vitro diagnostic market for early diagnosis of disease. Among them, methods using nucleic acids are usefully used for diagnosing causative genetic factors caused by infection by viruses and bacteria based on high specificity and sensitivity.

Most of the diagnostic methods using nucleic acids include methods using nucleic acid amplification reaction amplifying target nucleic acid (e.g., viral or bacterial nucleic acid). As a representative example, polymerase chain reaction (PCR) among nucleic acid amplification reactions includes repeated cycles of denaturation process of double-stranded DNA, annealing process of oligonucleotide primers to a DNA template, and extension process of primers by DNA polymerase (Mullis et al., U.S. Pat. No. 4,683,195, 4,683,202 and 4,800,159; Saiki et al., Science 230:1350-1354 (1985)).

PCR-based technologies are widely used in scientific applications or methods in biological and medical research fields as well as amplification of target DNA sequences, such as reverse transcription PCR (RT-PCR), differential display PCR (DD-PCR), cloning of known or unknown genes by PCR, rapid amplification of cDNA ends (RACE), arbitrarily primed PCR (AP-PCR), multiplex PCR, SNP genome typing, and PCR-based genomic analysis (McPherson and Moller, (2000) PCR. BIOS Scientific Publishers, Springer-Verlag New York Berlin Heidelberg, NY).

Other methods for amplifying nucleic acids include following methods: Ligase Chain Reaction (LCR), Strand Displacement Amplification (SDA), Nucleic Acid Sequence-Based Amplification (NASBA), Transcription Mediated Amplification (TMA), Recombinase Polymerase Amplification (RPA), Loop-mediated isothermal amplification (LAMP), and Rolling-Circle Amplification (RCA)).

As described above, molecular diagnostics technologies for detecting a target nucleic acid in one tube based on a nucleic acid amplification reaction has been used. Recently, multiplex diagnosis technologies for detecting a plurality of target nucleic acids in one tube is being used. As a representative example, multiplex PCR among PCR-based technologies means a technology amplifying and detecting a plurality of regions in a plurality of target nucleic acid molecules simultaneously by using a combination of a plurality of oligonucleotide sets (e.g., forward and reverse primers, and probes) in one tube.

The oligonucleotide set used in the molecular diagnostic technique should be designed to have performance capable of detecting a plurality of nucleic acid sequences of a target nucleic acid molecule with maximum coverage. In order for oligonucleotides (primers and probes) included in the oligonucleotide set to be designed to have the ability to detect a target, sufficient information about the nucleic acid sequence of the target nucleic acid molecule needs to be provided.

Accordingly, a technology of collecting information about nucleic acid sequences of target nucleic acid molecules in various ways and applying the information to the design of oligonucleotides is being used.

As a representative example, there is a technique for obtaining sequence information registered as the nucleic acid sequence of a target nucleic acid molecule in a public sequence database and using it to design oligonucleotides for detecting the target nucleic acid molecule. The public sequence database may include information about a genome sequence or a gene sequence of a specific organism, a host of the organism, and the like, as an experiment result obtained by a research institute, a business, an individual, and the like. As such, the sequence information registered in the public sequence database may be used in a design process of oligonucleotides used for detection of a target pathogen that causes infection in a specific host.

As an example, in the design process of an oligonucleotide for detecting Rotavirus that causes diarrhea, among Rotavirus A to H species belonging to the Rotavirus family, sequence information of Rotavirus A to C species causing infection to a Homo sapiens target may be selectively used. The oligonucleotide designed by using the above information may efficiently function as a molecular diagnostic reagent for detecting whether a genetic material of Rotavirus is present in a sample taken from a human.

However, even though sufficient information on the nucleic acid sequence of the target nucleic acid molecule is provided for the design of the oligonucleotide, when inaccurate nucleic acid sequence information is included in the information, the design of the oligonucleotide may be made relatively inaccurate, and accordingly, the possibility of failing to accurately detect the target nucleic acid molecule increases.

While there is a possibility that inaccurate information is registered in the public sequence database due to human error such as an experimental error or a description error, there is a problem in that a technical means for verifying these errors is insufficient. In particular, in the case of host entity information registered together with a nucleic acid sequence, it is difficult to verify whether there is an error, which leads to a decrease in data reliability. In addition, when there is an error in the pathogen individual information registered together with the nucleic acid sequence, there is a problem in that the accuracy of the oligonucleotide design is greatly reduced, and thus the possibility of failure in accurate detection of the target nucleic acid molecule is further increased.

In the above-described example, if a sequence of Rotavirus E, which causes infection in Sus scrofa, is included in the information registered as the nucleic acid sequence of Rotavirus species A or C due to an error in the registration by the registrant, more oligonucleotides are consumed for unnecessary target detection. In addition, this causes a problem in that the accuracy of the molecular diagnostic reagent is reduced and the efficiency of the oligonucleotides is reduced.

### [Disclosure]

### [Technical Problem]

An object to be solved according to an embodiment is to solve the above-described problems, and includes providing a method for assuming an organism carrying a nucleic acid sequence or a host of the organism when the nucleic acid sequence is given.

In addition, the object may include a method for allowing the nucleic acid sequences whose organism or host information has been verified through the assumption of such an organism or host to be used in the development of molecular diagnostic reagents.

However, objects of the present disclosure are not limited to the foregoing, and other unmentioned objects would be apparent to one of ordinary skill in the art from the following description.

### [Technical Solution]

Disclosed is a computer-implemented method performed by a computer device using a memory, a processor, and one or more programs stored in the memory and configured to be executed by the processor, according to an embodiment of the present disclosure. The computer-implemented method may include: accessing an assumption model obtained by fine-tuning a pre-learned model; providing a nucleic acid sequence to the assumption model; and assuming an organism carrying the nucleic acid sequence or a host of the organism from the assumption model.

In an embodiment, the assumption may include assuming a category located in any one hierarchical level among a plurality of hierarchical levels constituting a biological classification system, wherein the category is a biological category of the organism or the host.

In an embodiment, the any one hierarchical level may be a species level in the biological classification system.

In an embodiment, the pre-trained model may use a plurality of nucleic acid sequences as a training data.

In an embodiment, the pre-trained model may be trained by a semi-supervised learning method performed in such a manner that a mask to some of bases in the nucleic acid sequences is applied and then an answer to the masked base is made right.

In an embodiment, the pre-trained model may be trained by using nucleic acid sequences tokenized with tokens each having two or more bases.

In an embodiment, the tokens each may include bases tokenized by (i) dividing the nucleic acid sequence by k unit (k is a natural number) or (ii) dividing the nucleic acid sequence by a function unit.

In an embodiment, the fine-tuning may be performed by using a plurality of training data sets, and each training data set may include (i) a training input data including a nucleic acid sequence and (ii) a training answer data including a label data as to an organism carrying the nucleic acid sequence or a host of the organism.

In an embodiment, the fine-tuning may include (i) tokenizing the nucleic acid sequence included in the training input data to obtain a plurality of tokens, (ii) assuming the organism or the host of the nucleic acid sequence included in the training input data by using a context vector generated from the plurality of tokens, and (iii) training the pre-trained model in such a manner that a difference between the assumed result and the training answer data is reduced.

In an embodiment, the nucleic acid sequence provided to the assumption model may have a count of bases either not less than a preset first cutoff or not greater than a preset second cutoff.

In an embodiment, the method may further include, when the count of bases included in the nucleic acid sequence exceeds a preset second cutoff, obtaining one or more partial sequences from the nucleic acid sequence in such a manner that a count of bases not greater than the second cutoff are included, and wherein the providing the nucleic acid sequence may include providing the one or more partial sequences to the assumption model.

In an embodiment, the providing the nucleic acid sequence to the assumption model may include: providing to the assumption model one partial sequence including a count of bases not greater than the second cutoff when counted from a preset start point and not providing the remaining sequence excluding the one partial sequence to the assumption model; or providing to the assumption model each of a plurality of partial sequences including a count of bases not greater than the second cutoff.

In an embodiment, the assumption may include assuming an organism or a host for each of the plurality of partial sequences when the number of the partial sequence is plural, the method may further include statistically processing the assumption results of the organism or the host for each of the plurality of partial sequences, and the organism or the host assumed finally may be obtained by using the statistically processing result.

In an embodiment, the statistical processing may include at least one of a Majority vote method, a mean method, and a standard deviation method.

In an embodiment, (i) the assumed organism or the assumed host and (ii) the nucleic acid sequence may be used for a development of a molecular diagnostic reagent targeting the host.

In an embodiment, the development of the molecular diagnostic reagent may include a development of at least one of a primer and a probe used for detection of the organism.

In an embodiment, the providing the nucleic acid sequence may include obtaining the nucleic acid sequence from a sequence-related information including the nucleic acid sequence and an information about the organism or the host, and the method may further include obtaining a comparison result between the information about the organism and the assumed organism or a comparison result between the information about the host and the assumed host.

In an embodiment, the providing the nucleic acid sequence may include obtaining the nucleic acid sequence from a sequence-related information including the nucleic acid sequence and an information about the organism or the host, and the method may further include controlling in such a manner that the information about the organism or the host is modified to the assumed organism or the assumed host when the information about the organism or the host is different from the assumed organism or the assumed host.

In an embodiment, the nucleic acid sequence provided to the assumption model may have a count of bases not less than a preset first cutoff or not greater than a preset second cutoff, and the method may further include updating the first cutoff or the second cutoff to a different value when a count of the assumed organisms or the assumed hosts is not less than a preset first count or not greater than a second count.

In an embodiment, the updating may include: updating the first cutoff to a larger value when the count of the assumed organisms or the assumed hosts is not less than the first count, and updating the second cutoff to a smaller value when the count of the assumed organisms or the assumed hosts is not greater than the second count.

In an embodiment, the computer-implemented method may further include: controlling in such a manner that the nucleic acid sequence is not used for a development of a molecular diagnostic reagent targeting the organism or the host, when a count of the assumed organisms or the assumed host is not less than a preset reference count.

Disclosed is a computer program according to an embodiment of the present disclosure, stored in a computer-readable recording medium, programmed to perform each step included in the method.

Disclosed is a computer-readable recording medium according to an embodiment of the present disclosure, storing a computer program programmed to perform each step included in the method.

Disclosed is a computer device according to an embodiment of the present disclosure. The computer device may include: a memory configured to store at least one instruction; and the at least one instruction, when executed by the processor, cause the processor to: access an assumption model obtained by fine-tuning a pre-learned model; provide a nucleic acid sequence to the assumption model; and assume an organism carrying the nucleic acid sequence or a host of the organism from the assumption model.

Disclosed is a computer-implemented method according to an embodiment of the present disclosure, performed by a computer device using a memory, a processor, and one or more programs stored in the memory and configured to be executed by the processor. The computer-implemented method may include: accessing an assumption model obtained by fine-tuning a pre-learned model; providing a nucleic acid sequence to the assumption model; and assuming an organism carrying the nucleic acid sequence or a host of the organism from the assumption model, wherein the pre-trained model uses a plurality of nucleic acid sequences as a training data, and wherein the fine-tuning is performed by using a plurality of training data sets, and each training data set includes (i) a training input data including a nucleic acid sequence and (ii) a training answer data including a label data for an organism carrying the nucleic acid sequence or a host of the organism.

In an embodiment, the fine-tuning may include (i) tokenizing the nucleic acid sequence included in the training input data to obtain a plurality of tokens, (ii) assuming the organism or the host of the nucleic acid sequence included in the training input data by using a context vector generated from the plurality of tokens, and (iii) training the pre-trained model in such a manner than a difference between the assumed result and the training answer data is reduced.

Disclosed is a computer-implemented method according to an embodiment of the present disclosure, performed by a computer device using a memory, a processor, and one or more programs stored in the memory and configured to be executed by the processor. The computer-implemented method may include: obtaining a pre-trained model; and by fine-tuning the pre-trained model, obtaining an assumption model learned to assume an organism carrying a nucleic acid sequence or a host of the organism when the nucleic acid sequence is provided, wherein the fine-tuning is performed by using a plurality of training data sets, and each training data set includes (i) a training input data including a nucleic acid sequence and (ii) a training answer data including a label data for an organism carrying the nucleic acid sequence or a host of the organism.

In an embodiment, the label data may be a label data for a category located in any one hierarchical level among a plurality of hierarchical levels constituting a biological classification system, wherein the category may be a biological category of the organism or the host.

In an embodiment, the pre-trained model may use a plurality of nucleic acid sequences as a training data.

In an embodiment, the pre-trained model may be trained by a semi-supervised learning method performed in such a manner that a mask to some of bases in the nucleic acid sequences is applied and then an answer to the masked base is made right.

In an embodiment, the pre-trained model may be trained by using a nucleic acid sequence tokenized with tokens each having two or more bases.

In an embodiment, the tokens each may include bases tokenized by (i) dividing the nucleic acid sequence by k unit (k is a natural number) or (ii) dividing the nucleic acid sequence by a function unit.

In an embodiment, the fine-tuning may include (i) tokenizing the nucleic acid sequence included in the training input data to obtain a plurality of tokens, (ii) assuming the organism or the host of the nucleic acid sequence included in the training input data by using a context vector generated from the plurality of tokens, and (iii) training the pre-trained model in such a manner that a difference between the assumed result and the training answer data is reduced.

In an embodiment, the assumption model in the obtaining the assumption model may be learned to: assume an organism or a host of one or more partial sequences when the one or more partial sequences are provided, wherein the one or more partial sequences are obtained from the nucleic acid sequence in such a manner that include a count of bases not less than a preset second cutoff when a count of bases included in the nucleic acid sequence exceeds the second cutoff.

In an embodiment, the assumption model in the obtaining the assumption model may be learned to: assume an organism or a host for each of a plurality of partial sequences when the number of the partial sequences is plural, statistically process the assumption results of the organism or the host for each of the plurality of partial sequences, and finally assume the organism or the host of the nucleic acid sequence by using the statistical processing result.

### [Advantageous Effects]

According to an embodiment of the present disclosure, an organism carrying a nucleic acid sequence or a host of the organism can be accurately assumed by using the nucleic acid sequence.

In addition, by using the assumption model trained by the transfer learning method, the accuracy of the assumption can be sufficiently secured even if the learning using a small amount of labeled training data is performed. Further, even if there is no additional information on the target organism or the host of the organism, it is possible to accurately assume the organism carrying the nucleic acid sequence or the host of the organism by using only the corresponding nucleic acid sequence.

In addition, the nucleic acid sequence whose organism or host information is verified through the assumption of the organism or the host can be used for the development of molecular diagnostic reagents. For example, in the development of a reagent for molecular diagnostics targeting a specific host, a nucleic acid sequence in which information on the corresponding host is correctly described can be used. As another example, in the development of a molecular diagnostic reagent for detection of a specific organism, a nucleic acid sequence in which information on the corresponding organism is correctly described can be used. As described above, when the registered organism or host is inaccurate, the corresponding nucleic acid sequence is excluded from the development of the molecular diagnostic reagent, and thus the efficiency of oligonucleotide design can be improved. This may lead to improved detection accuracy for the target nucleic acid.

The effects of the present disclosure are not limited to the foregoing, and should be understood to include all effects that can be inferred from the configuration of the present disclosure described in the detailed description or claims of the present disclosure.

### [Description of Drawings]

FIG. 1 is a block diagram illustrating a computer device according to an embodiment.
FIG. 2 exemplarily illustrates a conceptual diagram of a process of pre-training according to an embodiment.
FIG. 3 is a view illustrating a structure and an operation of a BERT-based language model used in the pre-training process according to an embodiment.
FIG. 4 is a view illustrating an example method for predicting probabilities per base of the mased mases by the pre-trained model in the computer device according to an embodiment.
FIG. 5 exemplarily illustrates a conceptual diagram of a process of fine-tuning according to an embodiment.
FIG. 6 is a conceptual diagram illustrating the biological classification system and the biological category according to an embodiment.
FIG. 7 exemplarily illustrates a structure and an operation of a BERT-based assumption model in the process of fine-tuning according to an embodiment.
FIG. 8 exemplarily illustrates a process in which the nucleic acid sequence is preprocessed into partial sequences according to an embodiment.
FIG. 9 illustrates an example flow diagram for obtaining the assumption model in accordance with an embodiment
FIG. 10 is a conceptual diagram illustrating an inference operation of the assumption model according to an example embodiment.
FIG. 11 illustrates an example scheme in which the assumption model 1010 assumes the probability value per category according to an embodiment.
FIG. 12 illustrates an example flow diagram for assuming the organism or the host according to an embodiment.
FIG. 13 is a flowchart illustrating a process in which the computer device according to an embodiment allows the assumption result by an assumption model to be used for development of a molecular diagnostic reagent.
FIG. 14 illustrates an exemplary method in which the computer device controls the assumption result obtained by the assumption model to be used for the development of the molecular diagnostic reagent according to an embodiment.

### [Mode for Invention]

Various exemplary embodiments are described with reference to the drawings. In the present specification, various descriptions are presented for understanding the present disclosure. Prior to describing the specific content for carrying out the present disclosure, it should be noted that the configurations that are not directly related to the technical gist of the present disclosure are omitted within the scope of not disturbing the technical gist of the present disclosure. Further, the terms or words used in the present specification and the claims should be interpreted to have meanings and concepts consistent with the technical spirit of the present disclosure based on the principle that the inventor can define the concept of an appropriate term to best describe the invention.

A term "or" is intended to refer to not exclusive "or", but inclusive "or". That is, when it is not specified or unclear on the context, "X uses A or B" is intended to mean one of natural inclusive substitutions. Further, it should be understood that the term "and/or" used in this specification designates and includes all available combinations of one or more items among listed related items.

A term "comprise" and/or "comprising" is understood that the corresponding feature and/or component are present. However, it should be understood that a term "include" and/or "including" does not preclude existence or addition of one or more other features, constituent elements and/or a group of them. Further, when it is not separately specified or it is not clear from the context to indicate a singular form, the singular form in the specification and the claims is generally interpreted to represent "one or more".

In addition, a term "providing" and/or "provision" may be interpreted to include a process for obtaining specific information by a subject or directly or indirectly transmitting and receiving the specific information to the specific target, and to comprehensively include the performance of related operations required in this process.

Description of the suggested exemplary embodiments is provided to allow those skilled in the art to use or embody the present disclosure. Various modifications to these embodiments may be apparent to those skilled in the art. Generic principles defined herein may be applied to other embodiments without departing from the scope of the present disclosure. Therefore, the present disclosure is not limited to the embodiments suggested herein. The present disclosure needs to be interpreted within the broadest scope consistent with principles suggested herein and novel features.

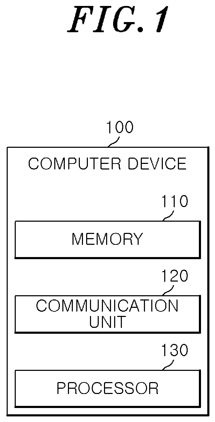

Prior to describing FIG. 1, terms used herein are described.

In the disclosure, the term "target nucleic acid sequence" refers to a target analyte (e.g., a target nucleic acid molecule) indicated as a specific nucleic acid sequence. In addition, the nucleic acid sequence means that bases are arranged in order, wherein the base is one of the components of a nucleotide. For example, the nucleic acid sequence can be used interchangeably herein with the base sequence. Each of the individual bases constituting a nucleic acid sequence may correspond to one of four types of bases, for example, adenine (A), guanine (G), cytosine (C), and thymine (T).

In the disclosure, the term "analyte" may refer to a variety of substances (e.g., biological and non-biological substances). Specifically, the target analyte may include the biological substance, more specifically at least one of nucleic acid molecules (e.g., DNA and RNA), proteins, peptides, carbohydrates, lipids, amino acids, biological compounds, hormones, antibodies, antigens, metabolites, and cells.

In the disclosure, the term "target analyte" or "organism" may include a living thing or an organism in any form to be analyzed, obtained, or detected. For example, the organism may refer to an organism that belongs to one genus, species, subspecies, subtype, genotype, serotype, strain, isolate, or cultivar. In the disclosure, "organism" can be used interchangeably with "target analyte".

Examples of the organism include prokaryotic cells (e.g., Mycoplasma pneumoniae, Chlamydophila pneumoniae, etc.), eukaryotic cells (e.g., protozoa and parasites, fungi, yeasts, higher plants, lower animals, and higher animals including mammals and humans), viruses, or viroids. Examples of the parasites of the prokaryotic cells include Giardia lamblia, Entamoeba histolytica, etc. Examples of the viruses include influenza A virus (Flu A), influenza B virus (Flu B), respiratory syncytial virus A (RSV A), respiratory syncytial virus B (RSV B), Covid-19 virus, parainfluenza virus 1 (PIV 1) to parainfluenza virus 4 (PIV 4), human rhinovirus (HRV), coronavirus, and adenovirus. The virus may include various other known viruses, and is not limited to the above-described examples.

The organism in the present disclosure may include not only the above-described viruses, but also various analytes such as bacteria and human, and the scope for the organism is not limited to the above-described examples.

In the present disclosure, "organism carrying nucleic acid sequence" means an organism represented by a nucleic acid sequence when the nucleic acid sequence is given. For example, when the result of sequentially listing bases, which are one of components of nucleotides included in the nucleic acid molecule of a specific organism, corresponds to the result of sequentially listing bases according to the given nucleic acid sequence, the specific organism may be considered to be an organism carrying the corresponding nucleic acid sequence. As another example, an organism carrying a nucleic acid sequence refers to an organism (e.g., a pathogen) having a genetic material corresponding to the nucleic acid sequence. The type of organism carrying such a nucleic acid sequence may be one or more according to embodiments, and for example, the type may be expressed in different names and/or numbers depending on which hierarchical level on the biological classification system the biological category corresponding to the type is set to be located.

In the present disclosure, "biological taxonomy hierarchy" is a classification system for distinguishing a range to which an organism belongs. The biological classification system according to an embodiment may be constituted of a plurality of hierarchical levels. For example, the plurality of hierarchical levels represented by Species, Genus, Family, Order, Class, Phylum, Kindom, or Domain, etc., may be included within the scope of the biological classification system in the present disclosure. As an example, the biological classification system according to an embodiment of the present disclosure may have a hierarchical structure. Here, the hierarchical structure may mean a hierarchical structure that is a biological system structure in which an upper hierarchical level encompasses a lower hierarchical level.

In the hierarchical structure, the upper hierarchical level may include all components included in the lower hierarchical level. For example, the upper hierarchical level for the lower hierarchical level including the components of A, B, and C may include at least the components of A, B, and C and may further include additional components.

In the present disclosure, "reagent for molecular diagnostics" means a reagent used for detection of a target analyte. For example, the molecular diagnostic reagent may include one or more oligonucleotides used in an amplification reaction for amplifying a target analyte or a signal indicating the presence of the target analyte.

In the disclosure, the term "oligonucleotide" refers to a linear oligomer of natural or modified monomers or linkages. The oligonucleotide includes deoxyribonucleotides and ribonucleotides, can specifically hybridize with a target nucleotide sequence, and is naturally present or artificially synthesized. The oligonucleotide of the present invention may include naturally occurring dNMPs (i.e., dAMP, dGMP, dCMP and dTMP), nucleotide analogs, or derivatives. In particular, the oligonucleotide is a single strand composed of a deoxyribonucleotide. The oligonucleotide includes oligonucleotides that hybridize with cleavage fragments which occur depending on a target nucleic acid sequence. Especially, the oligonucleotide includes a primer and/or a probe.

In the disclosure, the term "primer" refers to an oligonucleotide that can act as a point of initiation of synthesis under conditions in which synthesis of primer extension products complementary to a target nucleic acid strand (a template) is induced, i.e., in the presence of nucleotides and a polymerase, such as DNA polymerase, and under appropriate temperature and pH conditions.

In the disclosure, the term "probe" refers to a single-stranded nucleic acid molecule containing a portion or portions that are complementary to a target nucleic acid sequence. The probe may also contain a label capable of generating a signal for target detection.

Meanwhile, the molecular diagnostic reagent according to an embodiment may be a reagent used to detect a target analyte, but targeting a specific host. For example, as the molecular diagnostic reagent is put into a reaction vessel together with a sample (e.g., cell, blood, saliva, or swap) collected from host, the molecular diagnostic reagent may be used in a subsequent process in such a manner that an amplification reaction of the target analyte or a signal indicating the presence of the target analyte is performed to detect the target analyte. As an example, the molecular diagnostic reagent for detecting Rotavirus that causes diarrhea may include a primer and/or a probe used to detect whether a genetic material of Rotavirus is present in a sample collected from a human host.

In the disclosure, the term "host" refers to an entity corresponding to a host of an organism. The meaning of the host according to an embodiment may be defined in association with a nucleic acid sequence, and for example, a host of a nucleic acid sequence means the host of an organism carrying the corresponding nucleic acid sequence.

The host according to an embodiment may refer to an object from which the corresponding nucleic acid sequence is sampled. As an example, the host refers to a subject who provides a sample containing a nucleic acid sequence of a target analyte, and as the sample is analyzed by a researcher and information about the nucleic acid sequence is obtained, information about the nucleic acid sequence of the target analyte, information about the target analyte (organism), and information about the host may be recorded together. For example, when a nucleic acid sequence of Rotavirus A species is detected in a sample taken from a human nasopharynx, the target analyte (organism) of the nucleic acid sequence will be Ratavirus, and the host of the nucleic acid sequence will be a human (e.g. Homo sapiens).

The host according to another embodiment may comprehensively mean an entity known as host of an organism carrying the corresponding nucleic acid sequence. For example, the host may be a known entity that can be in a parasitic or symbiotic relationship with the organism containing the nucleic acid sequence. As another example, the host may be an entity recorded by a registrant as corresponding to a host of the organism carrying the nucleic acid sequence.

The host according to an embodiment of the present disclosure may be any living organism in which an organism may be parasitic or symbiotic, and may be, for example, a human, a plant, and an animal, but is not limited thereto. According to an embodiment, the living organism that may be a host may include some of the various examples of the above-described organism.

FIG. 1 is a block diagram illustrating a computer device according to an embodiment.

The computer device 100 according to an embodiment of the present disclosure may include a memory 110, a communication unit 120 and a processor 130.

The configuration of a computer device 100 illustrated in FIG. 1 is merely a simplified example. In an embodiment, the computer device 100 may include other configurations for performing a computing environment of the computer device 100, and only some of the disclosed configurations may also configure the computer device 100.

The computer device 100 may mean a node configuring a system for implementing exemplary embodiments of the present disclosure. The computer device 100 may mean a predetermined type of user terminal or a predetermined type of server. The foregoing components of the computer device 100 are illustrative, and some may be excluded, or additional components may be included. For example, when the computer device 100 includes a user terminal, an output unit (not illustrated) and an input unit (not illustrated) may be included within the range.

The memory 110 may store at least one instruction executable by the processor 130. In an embodiment, the memory 110 may store a predetermined type of information generated or determined by the processor 130 and a predetermined type of information received by the computer device 100. According to the exemplary embodiment of the present disclosure, the memory 110 may be a storage medium storing computer software that allows the processor 130 to perform operations according to the exemplary embodiment of the present disclosure. Therefore, the memory 110 may also mean computer readable media for storing software codes required for performing the exemplary embodiments of the present disclosure, data that is the execution target of the code, and a result of the code execution.

In an embodiment, the memory 110 may refer to any type of storage medium. For example, the memory 110 may include at least one type of flash memory, hard disk, multimedia card micro, card type memory (e.g., SD or XD memory etc.), Random Access Memory (RAM), Static Random Access Memory (SRAM), Read-Only Memory (ROM), Electrically Erasable Programmable Read-Only Memory (EEPROM), Programmable Read-Only Memory (PROM), magnetic memory, magnetic disk, and optical disk. The computer device 100 may operate in relation to a web storage performing a storage function of the memory 110 on the Internet. The descriptions of the foregoing memory are merely examples, and the memory 110 used in the present disclosure is not limited to the above examples.

The communication unit 120 may be configured regardless of its communication aspect, and may be configured of various communication networks, such as a Personal Area Network (PAN) and a Wide Area Network (WAN). Further, the communication unit 120 may be operated based on the publicly known World Wide Web (WWW), and may also use a wireless transmission technology used in PAN, such as Infrared Data Association (IrDA) or Bluetooth. For example, the communication unit 120 may take in charge of transmitting and receiving data required to perform a technique according to an embodiment of the present disclosure.

The processor 130 may perform technical features according to embodiments of the present disclosure described below, by executing at least one instruction stored in the memory 110. In an embodiment, the processor 130 may consist of one or more cores, and may include a processor for analyzing and/or processing data, such as a Central Processing Unit (CPU), a General Purpose Graphics Processing Unit (GPGPU), and a Tensor Processing Unit (TPU) of the computer device 100.

The processor 130 may read a computer program stored in the memory 110 to assume an organism carrying a given nucleic acid sequence or a host of the organism by using an assumption model, according to an embodiment. Here, the assumption model refers to an Artificial Intelligence (AI) based model learned to assume an organism carrying the nucleic acid sequence or a host of the organism by using the nucleic acid sequence.

According to an embodiment, the processor 130 may perform an operation for learning of a neural network. The processor 130 may perform calculation for learning of the neural network, for example, processing of training input data in deep learning (DN), extraction of a feature from the input data, calculation of an error, and updating of a weight of the neural network using backpropagation. At least one of a CPU, a GPGPU, and a TPU of the processor 130 may process the learning of the network function. For example, both the CPU and the GPGPU may process the learning of the network function and data classification using the network function. Further, according to an exemplary embodiment of the present disclosure, processors of a plurality of computing devices are used together to process the learning of the network function and data classification using the network function. Further, the computer program executed in the computing device according to the exemplary embodiment of the present disclosure may be a CPU, GPGPU, or TPU executable program.

In the disclosure, the computer device 100 may include any type of user terminal and/or any type of server. The user terminal may include any type of terminal capable of interacting with a server or other computing device. The user terminal may include, for example, a cell phone, a smart phone, a laptop computer, a personal digital assistants (PDA), a slate PC, a tablet PC, and an ultrabook. The server may include, for example, any type of computing system or computing device, such as a microprocessor, a mainframe computer, a digital processor, a portable device, a device controller, and the like.

Hereinafter, embodiments of a basic structure that the assumption model may have as an Al-based model are briefly described first. Specific structures and learning methods of the assumption model will be described later.

The assumption model in the present disclosure may refer to any type of computer programs that operates based on a network function, artificial neural network and/or neural network. Throughout the specification, a model, a network function, and a neural network may be used to have the same meaning. In the neural network, one or more nodes connected through the link may relatively form a relation of an input node and an output node. In the neural network, a characteristic of the neural network may be determined in accordance with the number of the nodes and links and a correlation between the nodes and links, and a weight assigned to the links. The neural network may be configured to include a set of one or more nodes. A subset of nodes configuring the neural network may configure a layer.

A deep neural network (DNN) may refer to a neural network including a plurality of hidden layers in addition to the input layer and the output layer. When the deep neural network is used, latent structures of the data may be identified. The deep neural network may include a convolutional neural network (CNN), a recurrent neural network (RNN), auto encoder, a generative adversarial network (GAN), a restricted Boltzmann machine (RBM), a deep belief network (DBN), a Q network, a U network, and a Siamese network, a generative adversarial network (GAN), and a transformer. Description of the above-described deep neural networks is only an example, and the present disclosure is not limited thereto.

The neural network may be learned by at least one of supervised learning, unsupervised learning, semi supervised learning, self-supervised learning or reinforcement learning methods. The learning of the neural network may refer to a process of applying knowledge for the neural network to perform a specific operation to the neural network.

The neural network may be learned to minimize an error of the output. Training data is repeatedly input to the neural network during the learning of the neural network, an output of the neural network for the training data and an error of the target are calculated, and an error of the neural network is back-propagated from the output layer of the neural network to the input layer direction so as to reduce the error to update a weight of each node of the neural network. In the case of the supervised learning, training data labeled with a correct answer (that is, labeled training data) may be used for each training data, but in the case of the unsupervised learning, training data not labeled with a correct answer (that is, unlabeled training data) may be used for each training data. A variation of the connection weight of the nodes to be updated may vary depending on a learning rate. The calculation of the neural network for the input data and the backpropagation of the error may configure a learning epoch. The learning rate may be differently applied depending on the repetitive number of the learning epochs of the neural network. In addition, methods of increasing training data, regularization, a dropout method which omits some nodes of the network during the learning process, or use of batch normalization layers may be applied in order to prevent the overfitting.

In an embodiment, the assumption model may include at least a portion of a transformer. The transformer may comprise an encoder encoding embedded data and a decoder decoding encoded data. The transformer may have a structure that receives a series of data and outputs a series of data of different type through encoding and decoding steps. In an embodiment, the series of data may be processed into a form computable by the transformer. A process of processing the series of data into the form computable by the transformer may include an embedding process. Expressions such as a data token, an embedding vector, and an embedding token may refer to embedded data in a form that can be processed by the transformer.

In order for the transformer to encode and decode a series of data, encoders and decoders in the transformer may be processed using an attention algorithm. The attention algorithm refers to an algorithm that obtains a similarity of one or more keys for a given query, applies the obtained similarity to a value corresponding each key, and then calculates an attention value by weighted sum.

Various types of attention algorithms may be classified, according to a method of setting a query, a key, and a value. For example, when one attention algorithm obtains an attention by setting the query, the key and the value all the same, the one attention algorithm may refer to a self-attention algorithm. When one attention algorithm obtains an attention by reducing a dimension of an embedding vector and obtaining individual attention heads in order to process a series of input data in parallel, the one attention algorithm may refer to a multi-head attention algorithm.

In an embodiment, the transformer may include modules performing a plurality of multi-head self-attention algorithms or multi-head encoder-decoder algorithms. In an embodiment, the transformer may include additional components other than the attention algorithm, such as embedding, normalization, and SoftMax. A method for constructing the transformer using the attention algorithm may include a method disclosed in Vaswani et al., Attention Is All You Need, 2017 NIPS, which is incorporated herein by reference.

The transformer may be applied to various data domains such as embedded natural language, segmented image data, and audio waveforms to convert a series of input data into a series of output data. In order to convert data having various data domains into a series of data that can be input to the transformer, the transformer may perform embedding the data. The transformer may process additional data representing a relative positional relationship or phase relationship between a set of input data. Alternatively, vectors representing a relative positional relationship or phase relationship between input data may be additionally applied to a series of input data, and the series of input data may be embedded. In an example, the relative positional relationship between the series of input data may include word order in a natural language sentence, relative positional relationship of each segmented image, and temporal sequence of segmented audio waveforms, but is not limited to. A process for adding a data representing the relative positional relationship or phase relationship between the series of input data may refer to positional encoding.

In an embodiment, the assumption model may include a Recurrent Neural Network (RNN), a Long Short-Term Memory (LSTM) network, a Bidirectional Encoder Representations from Transformers (BERT), or a Generative Pre-trained Transformer (GPT).

In an embodiment, the assumption model may be learned by a transfer learning method. Here, the transfer learning refers to a learning method in which a pre-trained model having a first task is obtained by pre-training using a large amount of unlabeled training data in a semi-supervised learning method or self-learning method and a targeted model is implemented by fine-tuning the pre-trained model to be suitable for a second task using a labeled training data in a supervised learning method.

In an embodiment, the first task of the pre-training and the second task of the fine-tuning may be different. As a specific example, the first task may be for language modeling of a phenomenon of a sequence pattern similar to a kind of language represented in a nucleic acid sequence of a target analyte (e.g., a virus). Alternatively, the first task may be a general-purpose task using the nucleic acid sequence of the target analyte. Also, the second task may be a sub-task of the first task and may be to assume an organism carrying the nucleic acid sequence and/or to assume a host of the organism by using the nucleic acid sequence. For example, a pre-trained language model may be obtained by training the training data including nucleic acid sequences of various viruses by using a language model to achieve a task for language modeling the sequence patterns of viruses. And then, the assumption model may be implemented by fine-tuning a structure and weights of the pre-trained language model to achieve a task for assumption of the organism or the host.

In an embodiment, the assumption model learned in the transfer learning method may refer to a model in which an output of an organism assumption value of a host assumption value in each of the plurality of nucleic acid sequences is applied as the fine-tuning, after a model is pre-trained by using information about the types and order of bases. In an embodiment, the pre-trained model may refer to a deep-learning language model learned according to a specific task (e.g., classification, detection, segmentation, etc.) or a general-purpose task. For example, the pre-trained model may be pre-trained based on types of bases (e.g., A, G, C, and T) and the arrangement order of bases.

In an embodiment, the meaning of the fine-tuning may include the concept of transferring the pre-trained model to a specific task, such as organism assumption or host assumption, and then training the model post-trained. Specifically, the fine-tuning may mean taking a pre-trained model, modifying it to fit the task for assuming an organism or host, and updating weights of the pre-trained model. As an example, the fine-tuning may include a process in which parameters of the pre-trained model are updated by additionally training the pre-trained model by using specific datasets for organism assumption or host assumption.

Hereinafter, various embodiments of a process in which the assumption model presented above is learned by the transfer learning method will be described in more detail.

### Examples of Pre-Training

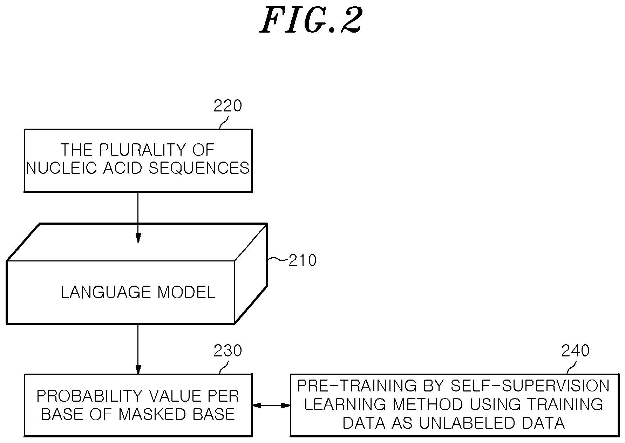

FIG. 2 exemplarily illustrates a conceptual diagram of a process of pre-training according to an embodiment. In an embodiment, FIG. 2 illustratively describes a method in which the pre-training is performed using a language model 210. A language model 210 performed pre-training may correspond to the pre-trained model in the present disclosure.

Referring to FIG. 2, the computer device 100 may perform the process of pre-training by using the language model 210. In an embodiment, the language model 210 is an artificial neural network, and may comprise at least a part of the above-described transformer. As a specific example, the language model 210 may include BERT or GPT, which are transformer-based language models.

In an embodiment, a plurality of nucleic acid sequences 220 may be used as a training data in the process of pre-training. Here, the plurality of nucleic acid sequences 220 refer to information about at least a part of nucleic acid sequences of an organism, and may mean, for example, information about the type and order of bases included in the genome of a pathogen or some of its genes, or information about the arrangement of the base pairs.

In one embodiment, the nucleic acid sequence 220 used in the pre-training process may not be limited to the type of organism carrying the nucleic acid sequence. For example, nucleic acid sequences of various organisms whose biological categories are not limited may be widely collected and used as training data. In another example, at least some of the nucleic acid sequences among the collected nucleic acid sequences may be selected randomly or according to preset rules (e.g., selecting a predetermined number of pieces of information by category) and used as training data. Here, the biological category is intended to express the position or range of each organism in the biological classification system, which will be described later.

In another embodiment, the nucleic acid sequence 220 used in the pre-training process may be partially limited to the type of organism carrying the nucleic acid sequence. Specifically, the plurality of nucleic acid sequences 220 may be obtained from organisms belonging to one or more preset biological categories. For example, nucleic acid sequences of viruses belonging to the Order level in the biological classification system (e.g., virus order) may be collected in large quantities and used as training data in the pre-training process.

In an embodiment, the plurality of nucleic acid sequences 220 may be obtained data obtained from a public database or data processed, modified, or separated therefrom. For example, the computer device 100 may access the public database such as National Center for Biotechnology Information (NCBI) or Global Initiative for Sharing All Influenza Data (GISAID), etc., collect a large amount of virus sequences registered in the public database, and perform a text preprocessing the collected virus sequences to process into training data for the pre-training.

In an embodiment, the language model 210 may be learned to predict a probability value 230 per base of masked base, based on a type and an order of bases in the plurality of nucleic acid sequences 220. Specifically, the language model 210 may be trained by a semi-supervised learning method or a self-supervised learning method performed in such a manner that a mask to some of bases in the nucleic acid sequences is applied and then an answer to the masked base is made right in the process of pre-training. For example, the language model 210 may be pre-trained by a self-supervision learning method 240 performed in which through a process of masking by randomly determining a base within a nucleic acid sequence, unlabeled training data is converted into labeled training data, and a task is assigned to get the answer to the masked base right by using the labeled training data.

For example, the language model 210 may assign a probability to a sequence of bases included in the plurality of nucleic acid sequences, consider which bases appear before and after the masked base, and output a probability value 230 per base by predicting an occurrence probability of multiple bases that can be a masked base candidate. In addition, the language model 210 may calculate an error by comparing the probability value 230 per base of the masked base, which is output data, and types and order of bases included in the plurality of nucleic acid sequences. Further, parameters of the language model 210 may be updated according to backpropagation for reducing errors.

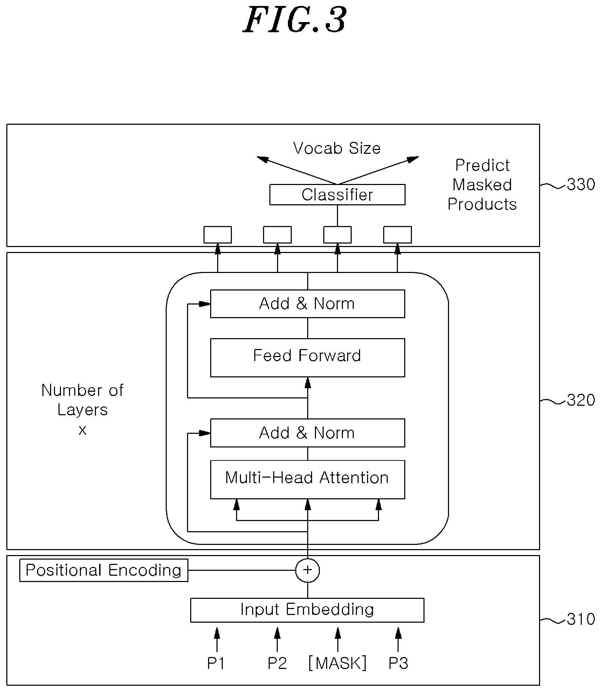

FIG. 3 is a view illustrating a structure and an operation of a BERT-based language model 210 used in the pre-training process according to an embodiment. As a specific embodiment, at least a part of BERT using a structure in which a plurality of encoders encoding embedded data are connected may be used for the pre-training using the language model 210.

Referring to FIG. 3, the language model 210 may refer to a classification model that outputs a plurality of prediction values for each of masked tokens using the masked tokens and non-masked tokens corresponding to the plurality of nucleic acid sequences 220. Here, one prediction value may correspond to one class of the language model 210. As an example, the language model 210 may receive the masked tokens and non-masked tokens as the nucleic acid sequences. As another example, the language model 210 may receive the nucleic acid sequences as input and perform preprocessing the input nucleic acid sequences to generate the masked tokens and non-masked tokens.

As shown in the FIG. 3, the language model 210 may include at least one of an input embedding layer 310, an encoder layer 320 and a first classifier layer 330.

In an embodiment, the input embedding layer 310 may convert the plurality of nucleic acid sequences 220, which are a series of input data, into a form computable by an encoder. In an embodiment, the input embedding layer 310 may include at least one of a token embedding layer for tokenizing bases in the nucleic acid sequences 220, and a position (or positional) embedding layer for applying a position data to vectors. In addition, according to embodiments, the input embedding layer 310 may further include an additional embedding layer such as a segment embedding layer, etc.

In an embodiment, the token embedding layer may perform a tokenization process that the nucleic acid sequences 220 are tokenized with tokens each having two or more bases. In an embodiment, the tokenization process may refer to an operation of grouping a plurality of bases included in the nucleic acid sequences. Each one of the tokens generated in the tokenization process may include one or more bases.

In an embodiment, the tokens may include each bases tokenized by (i) dividing the nucleic acid sequences by k unit (k is a natural number) or (ii) dividing the nucleic acid sequences by a function unit. For example of the former, a k-mer technique in which k bases are divided by k unit may be used in the tokenization process. In an example using the k-mer technique, if k is 3, the number of bases in each token may be three in total. For another example of the later, a gene prediction technique in which the nucleic acid sequences are divided according to a function for splicing may be used in the tokenization process. As a specific example, the dividing by the function unit may include at least one of dividing by codon unit capable of coding one amino acid and dividing by section unit related to such as gene expression (e.g., transcription, translation) or expression pattern.

In a further embodiment, the tokenization process may be performed based on various techniques. As an example, a tokenization of a nucleic acid sequence may be performed based on a Bite Pair Encoding algorithm. As another example, the nucleic acid sequence 220 may be tokenized based on an optimal k-mer size. In this example, since each organism may have a specific k-mer size that may best represent its genome, a tokenization may be performed based on the specific k-mer size determined in organism unit. In another example, a tokenization may be performed based on a DNA motif. As another example, a tokenization of the nucleic acid sequence 220 may be performed based on an exon, which is a unit of a coding region transcribing RNA within a gene of higher organisms.

In an embodiment, the token embedding layer may generate masked tokens and non-masked tokens by performing preprocessing a plurality of tokens generated through the tokenization process. For example, the token embedding layer may generate the masked tokens by masking at least one of a plurality of non-masked tokens tokenized from the nucleic acid sequence with a special token, called [MASK] token.

In an embodiment, the token embedding layer may represent each token as a vector, for example, may convert the tokenized bases into an embedding vector in form of a dense vector by word embedding the tokenized bases.

In an embodiment, the position (or positional) embedding layer may apply a position data to the embedding vectors before the embedding vectors are used as an input of an encoder. For example, a position embedding for obtaining the position data through learning may be used. For example, a method for learning a plurality of position embedding vectors corresponding to a length of a nucleic acid sequence and adding the corresponding position embedding vector to each embedding vector may be used.

Embedded data processed into a form computable by an encoder while passing through the above-described input embedding layer 310 may be provided as an input to the encoder layer 320 having a structure in which a plurality of encoders are stacked. Accordingly, the result of calculating data embedded in the first encoder in the encoder layer 320 is output toward the next encoder, and a context vector generated comprehensively considering the input embedded data may be output in the last encoder.

In an embodiment, the encoder layer 320 may include N (e.g., 12, 24, etc.) encoder blocks. A structure in which N encoder blocks are stacked means that a meaning of an entire input sequence is repeatedly constructed N times. The larger the number of encoder blocks, the better the semantic relationship between bases in a nucleic acid sequence may be reflected. N encoder blocks may be configured in a form in which the entire input sequence is recursively processed.

In an embodiment, each encoder block may output a weight-based calculation result for provided input using a multi-head attention algorithm. For example, each encoder block may output a concatenation which is a result of calculating an attention h time using different weight matrices and connecting them together. As a result, even small differences may lead to significant differences in results. Further, learning effect may be improved as inputs and processing results in each encoder block are calculated through normalization, residual connection, and feed-forward neural network.

In an embodiment, the first classifier layer 330 may process a result output from the encoder layer 320 into a form of data meaningful to the user. For example, the first classifier layer 330 may include a classifier that performs a classification function for performing the first task by using an embedding vector (e.g., context vector) output from the last encoder block of the encoder layer 320 as an input. For example, the classifier may include a SoftMax function for outputting the probability value 230 per base of the masked token by using an output embedding vector corresponding to a position of the masked token as an input.

An error calculation and a weight update may be performed by comparing an output of the first classifier layer 330 with the bases included in the nucleic acid sequence 220 of the training data. As such the pre-training proceeds, the pre-trained model may be obtained. Since the pre-training using a Masked Language Modeling (MLM) method for finding an answer of the masked base predicts the masked bases in consideration of bases located in bi-direction within a given nucleic acid sequence, prediction accuracy is high. Further, a pre-trained model with a better understanding of a pattern of nucleic acid sequences having characteristics similar to a kind of language may be implemented.

In an embodiment, the method for learning to get the answer to the masked base right and the method for learning to correct an incorrect base after replacing some bases with other bases may be used together in the process of the pre-training. In addition, Next Sentence Prediction (NSP) may be further used in the process of the pre-training. For example, the pre-training using both the MLM method and the NSP method may be performed.

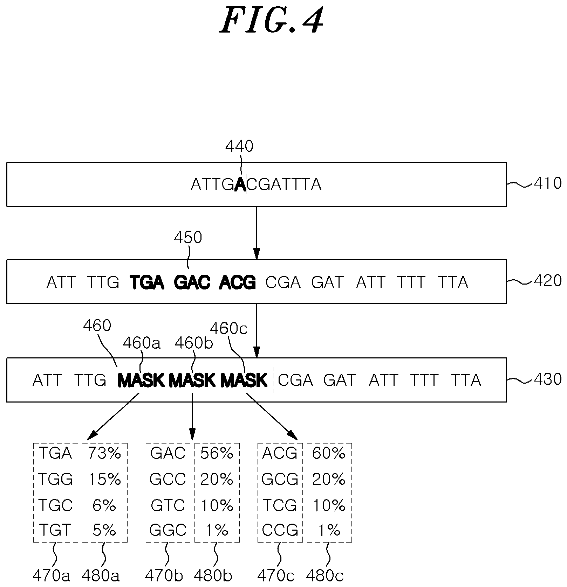

FIG. 4 is a view illustrating an example method for predicting probabilities 230 per base of the mased mases by the pre-trained model in the computer device 100 according to an embodiment.

FIG. 4 illustrates that the number of bases included in one token or one masked token is three. Depending on the implementation, it would be apparent to those skilled in the art that the number of bases in a token may include a various number.

In an embodiment, the pre-trained model may calculate the probability value 230 per base of a specific base 440 in the nucleic acid sequence 410. When the nucleic acid sequence 410 is provided to the pre-trained model, the pre-trained model may determine at least one specific base 440 from the nucleic acid sequence 410 and output the probability value 230 per base of the specific base 440. The nucleic acid sequence 410 may correspond to the nucleic acid sequence 220.

In an example shown in FIG. 4, the base 440 for calculating the probability value 230 per base in the nucleic acid sequence 410 is A. The pre-trained model may tokenize the nucleic acid sequence 410 consisting of a plurality of bases into a plurality of tokens 420.

FIG. 4 illustrates that each of the plurality of tokens 420 is generated according to a 3-mer technique. Each of the plurality of tokens 420 may include three bases. In an example in FIG. 4, a first base, A, in the nucleic acid sequence 410 may correspond to a token including ATT. A second base, T, in the nucleic acid sequence 410 may correspond to a token including ATT and a token including TTG. A third base, T, in the nucleic acid sequence 410 may correspond to a token including ATT, a token including TTG, and a token including TGA. A fourth base, G, may correspond to a token including TTG, a token including TGA, and a token including GAC. As such, FIG. 4 shows that tokens are generated while moving one base in the arrangement order of a plurality of bases in the nucleic acid sequence 410. In this example, two bases may be shared with each other for adjacent tokens.

In an embodiment, each of the generated tokens may correspond to a k-mer resulting from dividing bases in the nucleic acid sequence 410 by k unit. Here, k may be a natural number, for example, k may refer to a natural number not less than 3 and not more than 20. In this example, a number of bases in each of the tokens may correspond to k. That is, when k is 3, one token may include 3 bases.

In an embodiment, each of the masked tokens may include k bases. In addition, a count of the masked tokens generated corresponding to each of the bases in a range of kth to n-kth among the n bases comprised in the nucleic acid sequence may correspond to k. Here, each of k and n is a natural number, for example, k ≥ 2 and n ≥ 2k.

For example, among the plurality of tokens 420, the tokens 450 corresponding to the first base 440, A, of the nucleic acid sequence 410 may include TGA, GAC, and ACG. The tokens 450 may include a first token TGA including the first base 440 at a first position, a second token GAC including the first base 440 at a second position, and a third token ACG including the first base 440 A at a third position. In the example described above, since tokens are generated based on the 3-mer technique, one token may include three bases and a total of three tokens may correspond to one base.

In an embodiment, for the first base 440 of the plurality of bases 410, a first set of tokens 450 including the first base 440 at different positions and a first set of masked tokens 460 (460a, 460b, and 460c) corresponding to the first set of tokens 450 may be generated. In an embodiment, the probability value 230 per base of the first base 440 may be determined based on prediction values (480a, 480b, and 480c) output from the language model 210 for each of the first set of masked tokens 460 (460a, 460b, and 460c).

The pre-trained model may obtain the masked tokens 460 by applying a mask to the first set of tokens 450 that are at least some tokens of the plurality of tokens 420. For example, the pre-trained model may generate the masked tokens 460 by applying a mask to each of three tokens corresponding to the first base 440. In the example described above, since tokens are generated based on the 3-mer technique, one masked token may correspond to three bases, and a count of masked tokens may also correspond to three.

The pre-trained model may generate an intermediate input data 430 from the tokens 420. In an embodiment, the intermediate input data 430 may include the masked tokens 460 and non-masked tokens.

In an embodiment, the pre-trained model may obtain prediction values (480a, 480b, and 480c) of classes (470a, 470b, and 470c) for each of the masked tokens 460 (460a, 460b, and 460c). The pre-trained model may calculate the probability value 230 per base of the first base 440 based on the obtained prediction values (480a, 480b, and 480c).

In an embodiment, the computer device 100 may calculate the probability value 230 per base by using an average of the predicted values (480a, 480b, and 480c) for the classes (470a, 470b, and 470c). In another embodiment, the computer device 100 may calculate the probability value 230 per base for the first base 440 by using an weighted average of the predicted values (480a, 480b, and 480c) for each of the classes (470a, 470b, and 470c) based on the weights assigned to each of the classes (470a, 470b, and 470c).

In an embodiment, parameters of the pretrained model may be updated to minimize errors, by comparing the probability value 230 of the specific base 440 with the specific base 440 in the nucleic acid sequence 410, or by comparing prediction values (480a, 480b, 480c) of the masked tokens 460 (460a, 460b, and 460c) with tokens 450 corresponding to the specific base 440. In this way, the pre-trained model may be trained to get more accurate answer to the masked base.

### Examples of Fine-tuning

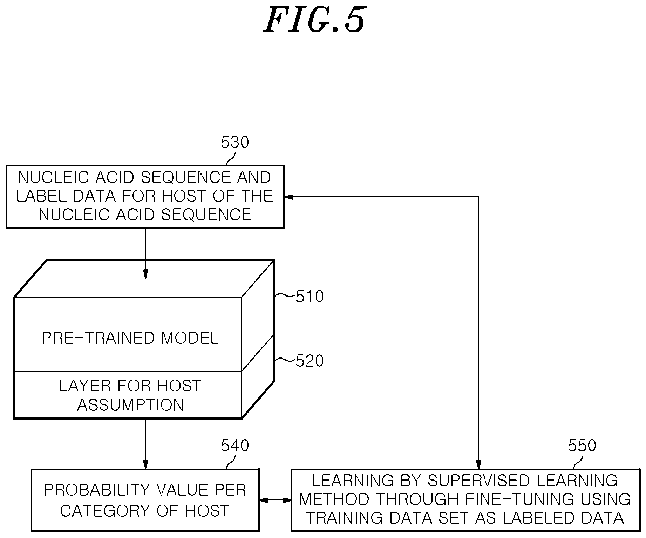

FIG. 5 exemplarily illustrates a conceptual diagram of a process of fine-tuning according to an embodiment. In an embodiment, the pre-trained model 510 illustrated in FIG. 5 may correspond to the language model 210 on which the pre-training is performed.

Referring to FIG. 5, an assumption model may be obtained by fine-tuning on the pre-trained model 510. In an embodiment, the process of fine-tuning may include a process of determining a structure of the assumption model by using the pre-trained model 510, and a process of training the assumption model with training data for host assumption or organism assumption. FIG. 5 illustrates the process of fine-tuning for assuming a host according to an embodiment, but the process of fine-tuning for assuming an organism according to another embodiment may be performed in a similar manner.

In an embodiment, the process of determining the structure of the assumption model using the pre-trained model 510 may be performed by applying a layer for host assumption or organism assumption to the pre-trained model 510. For example, in the case of fine-tuning for host assumption, one or more of the input embedding layer 310 and the encoder layer 320 may be obtained from the pre-trained model 510 on which weights are pre-calculated through the pre-training, a layer 520 for host assumption may be added to the last encoder block of the encoder layer 320. In the case of fine-tuning for organism assumption, the layer for organism assumption may be added in the same manner. In an embodiment, a layer for organism assumption may have at least partially the same structure as the layer 520 for host assumption, and for example, fine-tuning may be performed in such a manner that different training data sets are used while the structure of the model is the same as compared with host assumption.

In an embodiment, the fine-tuning may be performed by using a plurality of training data sets. Each training data set may include (i) a training input data including a nucleic acid sequence and (ii) a training answer data including a label data as to an organism carrying the corresponding nucleic acid sequence or a host of the organism. That is, in the fine-tuning for host assumption, each training data set may include a nucleic acid sequence and a label data 530 for host of the corresponding nucleic acid sequence. In addition, in the fine-tuning for organism assumption, each training data set may include a nucleic acid sequence and a label data for an organism of the corresponding nucleic acid sequence.

In an embodiment, the nucleic acid sequence used as the training input data in the process of fine-tuning may be limited in the type of organism carrying the corresponding nucleic acid sequence. Specifically, the nucleic acid sequence in the process of fine-tuning may be obtained from organisms belonging to one or more preset biological categories. For example, nucleic acid sequences of viruses belonging to the Order level in the biological classification system may be used as the training input data. As another example, nucleic acid sequences of a plurality of specific viruses (e.g., Rotavirus A, Influenza virus A, and Rabies lyssavirus) belonging to a species level in a biological classification system may be used as the training input data.

In an embodiment, the hierarchical level in which the organism carrying the nucleic acid sequence in the process of fine-tuning is located on the biological classification system may be a lower level than the hierarchical level in which the organism carrying the nucleic acid sequence 220 in the process of pre-training is located on the biological classification system. For example, when a large amount of virus sequences belonging to the Order level are collected in the process of pre-training and used as training data, nucleic acid sequences of organisms (e.g., Rotavirus A, Influenza virus A, Rabies lyssavirus) belonging to a predetermined biological category among virus sequences belonging to the species level lower than the Order level in the fine-tuning process may be used.

In an embodiment, the number of training data sets used in the process of fine-tuning may be limited to be within a preset range for each type of host or for each type of organism. For example, data sets within the number or the range determined for each biological category of host may be selected as the training data set in such a manner that the difference in the distribution of the count of training data sets for each host type is not greater than a preset level.

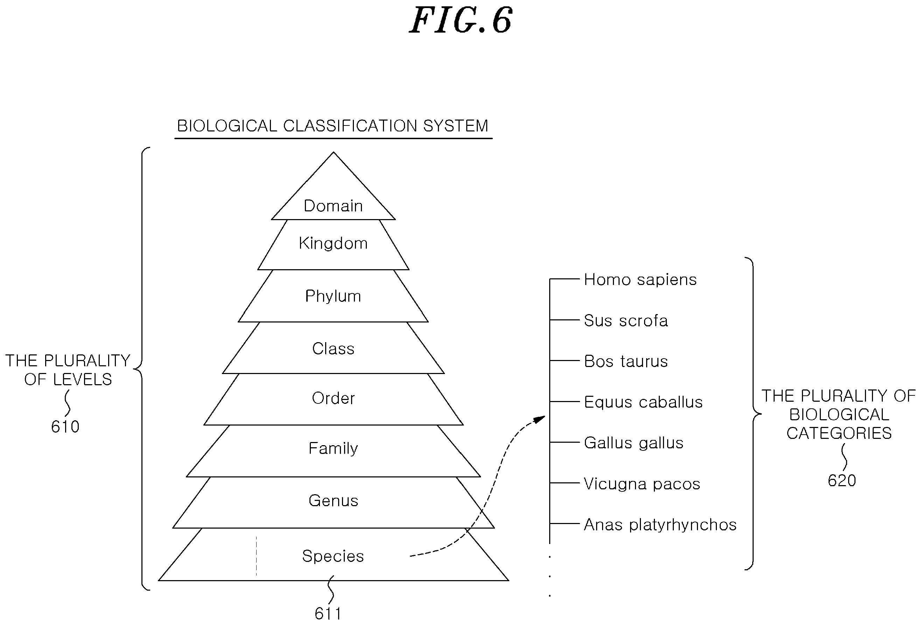

In an embodiment, the label data for host (or organism) used as the training answer data in the fine-tuning may mean label data for the type of host of an organism carrying the corresponding nucleic acid sequence (or the type of organism). Here, the type of host is a biological category of the host (or organism), and may include a category (e.g., a species name) located in any one hierarchical level among a plurality of hierarchical levels constituting a biological classification system. Here, the meaning of the biological category may be defined based on the biological classification system, and this will be described with reference to FIG. 6.

FIG. 6 is a conceptual diagram illustrating the biological classification system and the biological category according to an embodiment.

As illustrated in FIG. 6, the biological classification system is a classification system for classifying a range to which an organism belongs, and may be composed of a plurality of levels 610. The plurality of levels 610 may have a biological system structure in which an upper level encompasses a lower level, and may be expressed as at least one of levels including, for example, a Species, a Genus, a Family, an Order, a Alass, a Division, a Kingdom, and a Domain.

In an embodiment, the plurality of biological categories 620 may be located at any one of the plurality of levels 610. For example, the plurality of biological categories 620 may be located at a species level 611 in the biological classification system, and may be expressed by a species name of individuals, for example, Homo sapiens, Sus scrofa, Bos taurus, Equus caballus, and Gallus gallus, which may be a host of an organism.

FIG. 6 exemplarily shows some species as candidates that may be assumed as a biological category of host, but is not limited thereto, and various entities known as host of any organism may be applied. In addition, such a biological category may be interpreted in the same manner when referring to the type of organism carrying the corresponding nucleic acid sequence.

In an embodiment, the plurality of biological categories 620 are candidate groups of biological categories that may be assumed for host or an organism, and may be determined or classified based on the type of organism carrying the nucleic acid sequence. For example, when the type of the organism is a Rotavirus family, the plurality of biological categories 620 for host may include Homo sapiens, Sus scrofa, Bos taurus, Equus caballus, Gallus gallus, Vicugna pacos, and the like. As another example, when the type of the organism is an Influenza family, the plurality of biological categories 620 for host may include Homo sapiens, Sus scrofa, Anas platyrhynchos, Gallus gallus, Anas discors, Arenaria interpres, Anas acuta, Meleagris gallopavo, Anas clypeata, Equus caballus, Anas carolinensis, and the like. As another example, when the type of the organism is a Rabies lyssavirus family, the plurality of biological categories 620 for host may include Canis lupus, Bos taurus, Desmodus rotundus, Vulpes vulpes, Eptesicus fuscus, Mephitis mephitis, Homo sapiens, Procyon lotor, Felis catus, Tadarida brasiliensis, Vulpes lagopus, and the like.

Meanwhile, although the biological classification system having a hierarchical structure is illustrated in FIG. 6, but the present disclosure is not limited thereto. According to an embodiment, various classification systems such as a system group classification structure according to the characteristics of the classification group may be applied to the biological category.

Table 1 exemplarily shows N training data sets used for fine-tuning according to an embodiment. In Table 1, each training data set may include an input of a nucleic acid sequence (training input data) for each sample and a label data (training answer data) for a biological category of an organism carrying the nucleic acid sequence or a host of the organism. One biological category (e.g., a species name) may be mapped to one category identifier (e.g., M), and for example, the training answer data may include a category identifier mapped to the biological category of the corresponding host, as shown in Table 1.

**[Table 1]**

| | Nucleic acid sequence | Label data |
|---|---|---|
| Sample 1 | AGCATTGTGGGTAGTAAGGTATAAA AGCTCAAAATCTACA ... | 1 |
| Sample 2 | AGCGTTATTGTTGAGAAATGGATTG AGCACAAAAAAATTT ... | 2 |
| ... | ... | ... |
| Sample N | AGCTGTTTTTTTTTTTGTTGTGGGTAA AGCCTATAAATCC ... | M |

In an embodiment, the training data sets used for the fine-tuning may be a pre-verified data set. For example, nucleic acid sequences posted in biotechnology information (e.g., a paper, etc.) or registered in the public database such as NCBI or GISAID and information about the corresponding host and/or the corresponding organism may be collected through a predetermined medium or means. In addition, among the collected information, nucleic acid sequences verified that information about the host and/or the organism is correct by a preset verification method (e.g., manager verification, rule-based verification) may be selected as the training data sets.

In an embodiment, the number of training data sets used for the fine-tuning may be less than the number of training data used for the pre-training. For example, when hundreds of thousands to millions or more of large-scale training data are used for pre-training, about thousands to thousands of low- scale training data sets may be used for fine-tuning.

The assumption model may be fine-tuned in such a manner that the pre-trained model 510 outputs a probability value 540 per category of host by using the above-described training data sets. For example, in the process of fine-tuning for host assumption, the assumption model may output the probability value 540 per category of the host for the nucleic acid sequence, based on the type and order of bases included in each nucleic acid sequence that is training input data. In addition, a learning 550 may be performed by the supervised learning method through fine-tuning by using the training data set as the labeled data. Alternatively, in a similar manner to that described above, in the process of fine-tuning for assuming an organism, the assumption model may output the probability value 540 per category of the organism for the nucleic acid sequence, and learning may be performed in the supervised learning method by using the training data set.

For example, in the process of fine-tuning, the assumption model may output the probability value 540 per category as output data, which represents a probability value (e.g., 93%, 2%, 0.3%, etc.) that the biological category of host of the organism is each of the plurality of biological categories 620 (e.g., Homo sapiens, Sus scrofa, Bos taurus, etc.). In addition, the assumption model may calculate an error by comparing the output data with a biological category (e.g., Homo sapiens) labeled as a correct answer to each nucleic acid sequence, and update parameters of the assumption model according to backpropagation for reducing the error.

The fine-tuning according to an embodiment may include (i) tokenizing the nucleic acid sequence included in the training input data to obtain a plurality of tokens, (ii) assuming a host or an organism of the nucleic acid sequence included in the training input data by using a context vector generated from the plurality of tokens, and (iii) training the pre-trained model 510 in such a manner that a difference between the assumed result and the training answer data is reduced. Here, the training the pre-trained model 510 may correspond to implementing the trained assumption model by performing training on an assumption model having a structure including the pre-trained model 510 described above. In an example in which the assumption model includes the BERT, as a plurality of tokens are input to a plurality of encoders included in the BERT, a context vector in which a nucleic acid sequence is comprehensively considered may be output as one compressed vector representation. In addition, the context vector may be input to a classifier connected to the encoder, and a classification value or a probability value may be output from the classifier as a predicted result for the host or the organism. Furthermore, an error may be calculated by comparing the predicted result with label data for the host of label data for the organism included in the training answer data, and parameters of the assumption model may be updated to reduce the error.

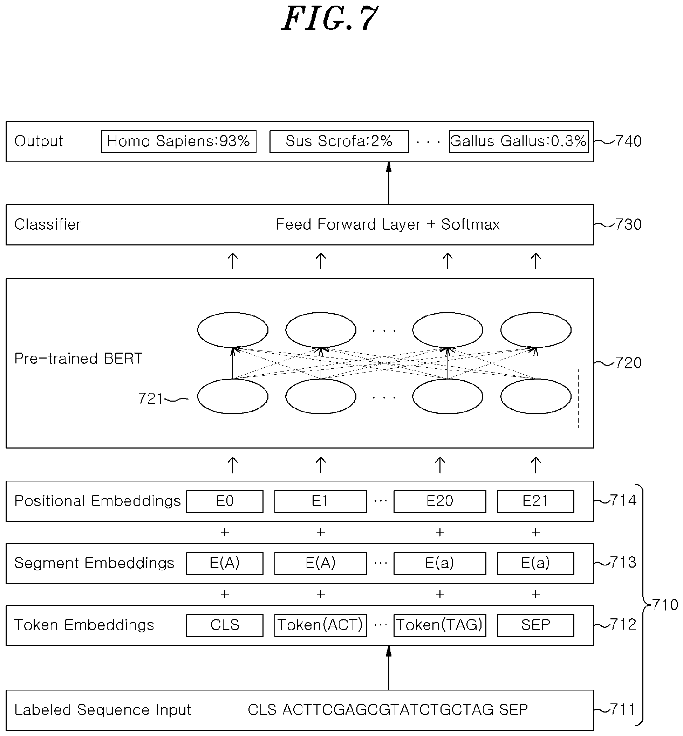

The above-described training process may be performed by using each of the plurality of training data sets, and more detailed descriptions thereof will be described with reference to FIG. 7.

FIG. 7 exemplarily illustrates a structure and an operation of a BERT-based assumption model in the process of fine-tuning according to an embodiment.

Referring to FIG. 7, the assumption model may include BERT. In an example in which the assumption model includes BERT, BERT may refer to a model in which supervised learning-based fine-tunning is applied to the pre-trained model 510 pre-trained by using semi-supervised learning. According to an example embodiment, the assumption model may include a derivative model of the BERT, such as ALBERT, RoBERTa, ELECTRA, and the like.

In an embodiment, the assumption model may include at least one of an input embedding layer 710, a pre-trained BERT 720, and a second classification layer 730.

In an embodiment, the pre-trained BERT 720 may include at least some of the layers of the pre-trained model 510. For example, the pre-trained BERT 720 applied to the assumption model may include the encoder layer 320 in which weights are pre-calculated by pre-training.

In an embodiment, the pre-trained BERT 720 may be obtained by at least partially modifying the pre-trained model 510. For example, the pre-trained BERT 720 applied to the assumption model may include the encoder layer 320 on which weights are pre-calculated by pre-training, and some of encoders in the encoder layer 320 may be freeze-processed in such a manner that weights are fixed. Accordingly, in the process of fine-tuning, the weights of the freeze layer 721 are fixed to the weight values in the pre-training, and weight values only for the remaining weights except for the freeze layer 721 may be updated through additional training. Accordingly, the learning time may be shortened, and the overfitting problem may be improved.

In an embodiment, the input embedding layer 710 may preprocess the input nucleic acid sequence and provide the preprocessed nucleic acid sequence to the pre-trained BERT 720. The input embedding layer 710 may convert a nucleic acid sequence, which is a series of input data, into a form that an encoder can compute. According to an embodiment, the input embedding layer 710 may correspond to the input embedding layer 310 of the pre-trained model 510.

In an embodiment, the input embedding layer 710 may include at least one of a labeled sequence input layer 711 for sequence embedding, a token embedding layer 712 for tokenization, a segment embedding layer 713 for segment information addition, and a positional (or position) embedding layer 714 for position information addition.

The labeled sequence input layer 711 may process an input nucleic acid sequence in a form suitable for embedding. For example, the labeled sequence input layer 711 may add a special classification token (CLS), which is a special token representing the start of the sequence, to the first position of the nucleic acid sequence, and may add a special classification token (SEP), which is a special token representing the end of the sequence, to the last position of the nucleic acid sequence.

The token embedding layer 712 may perform a tokenization process of obtaining a plurality of tokens by tokenizing the nucleic acid sequence. In an embodiment, the tokenization process performed by the token embedding layer 712 may include embodiments of the tokenization process described in the pre-training process. For example, the token embedding layer 712 may divide bases included in an input sequence output from the labeled sequence input layer 711 by using the k-mer technique to tokenize the divided bases, or may perform slicing by using a gene assumption technique of the function unit to tokenize the sliced bases. In addition, the token embedding layer 712 may process each token as a vector in the tokenization process.

The segment embedding layer 713 may process the plurality of tokens in such a manner that discrimination information for sequence discrimination is applied to the plurality of tokens. In an embodiment, when two or more partial sequences obtained from one nucleic acid sequence are input together, the segment embedding layer 713 may perform segment embedding by using different vector representations to discriminate each partial sequence. For example, when there are two input partial sequences, two vector representations may be used, the first vector (index 0) may be allocated to all tokens belonging to the first partial sequence, and the last vector (index 1) may be allocated to all tokens belonging to the second partial sequence. In another embodiment, when a nucleic acid sequence or a partial sequence is input to the assumption model one by one, the segment embedding layer 713 may be omitted or the same vector representation may be used.

The positional (or position) embedding layer 714 may apply position information to embedding vectors before a plurality of embedding vectors generated from the plurality of tokens through the above-described token embedding and segment embedding are used as inputs of the encoder.

The embedded data processed in the form computable by the encoder while passing through the above-described input embedding layer 710 may be input to the pre-trained BERT 720. The pre-trained BERT 720 may output a context vector by comprehensively considering the input embedded data.

In an embodiment, the second classification layer 730 may assume a biological category of the host or the organism by using a result output from the pre-trained BERT 720. For example, the second classification layer 730 may include a classifier performing a classification function for outputting a probability value 740 per category of the host by using an embedding vector(s) (e.g., context vector) output from the last encoder block of the pre-trained BERT 720 as an input. For example, the second classification layer 720 may output the probability value 540 per category of the host for each of the plurality of classes corresponding to the plurality of biological categories 620 by using the classifier, or may output the probability value 540 per category for one or more classes having the largest probability value or greater than a preset reference value among the plurality of classes.

In an embodiment, the second classification layer 730 may include a FC neural network and a SoftMax function for assuming a biological category of host or an organism. The second classification layer 730 may be configured to perform a classification function for outputting a result of the probability value 740 per category of the host or the probability value per category of the organism. For example, all embedding vectors output from the pre-trained BERT 720 may be input to the feed forward neural network in the FC structure, and the SoftMax function may be used as an active function of the output layer in the feed-forward neural network. A vector(s) of a specific dimension output from the neural network may have a real value between 0 and 1 while passing through the SoftMax function, and a total sum may be converted into a vector of 1, and the vector may be output as the probability value 740 per category.

In an embodiment, the probability value 740 per category of the host output from the second classification layer 730 or the probability value per category of the organism may be compared with label data for the biological category of the host or the organism that is the training answer data, and the assumption model may be additionally trained in such a manner that the error is minimized according to the comparison result.

Meanwhile, the count of bases in the nucleic acid sequence provided to the assumption model may be within a preset range. Specifically, the count of bases included in the nucleic acid sequence input to the assumption model may be not less than a preset first cutoff or not greater than a preset second cutoff. For example, the count of bases in a sequence input to the input embedding layer 710 of the assumption model may be not less than the preset first cutoff or not greater than the second cutoff, and may be not less than the first cutoff and not greater than the second cutoff.

In an embodiment, the first cutoff is a minimum reference value for the count of bases in the sequence input to the assumption model. For example, the first cutoff may be set to a value between 1,500 bp and 2,000 bp.

In an embodiment, the second cutoff is a maximum reference value for the count of bases in the sequence input to the assumption model. For example, the second cutoff may be set to a value between 10,000 bp and 20,000 bp.

In an embodiment, the first cutoff and/or the second cutoff may be changed based on whether the performance of the assumption model has achieved a minimum target level in the process of the fine-tuning. For example, when the assumption accuracy of the assumption model additionally trained using data sets of a preset ratio among the training data sets is not greater than the reference value, the first cutoff may be increased by a predetermined ratio to improve the assumption accuracy.

In an embodiment, when the type of the target organism is preset, the first cutoff and/or the second cutoff may be determined based on at least one of a length of a genome of the organism belonging to the type, a length of a reference sequence, the count of reference sequences, and diversity information. Here, the reference sequence refers to a nucleic acid sequence for representing the corresponding organism, and may include, for example, a base sequence first discovered for the type of the organism, or a base sequence occupying the largest proportion or more than a predetermined proportion. In addition, the diversity information may include, for example, a level of sequence difference regarding the difference between the reference sequence of the nucleic acid sequence and other sequences, and a range or number of non-conserved regions having a relatively high level of sequence difference in the nucleic acid sequence and conserved regions having a relatively low level of the same. For example, as the average count of bases of in the genome of viruses belonging to the target virus family increases, as the count of bases of a reference sequence increases, as the count of reference sequences decreases, as the range of the non-conservative region increases or the number of the region increases, as the range of the conservative region decreases or the number of region decreases, the first cutoff and/or the second cutoff may be set to a relatively large value. Accordingly, the assumption accuracy of the assumption model may increase.

In an embodiment, the second cutoff may be set based on a maximum value that can be input to the assumption model. For example, the second cutoff may be set in consideration of the maximum token size that may be calculated in the assumption model.

As described above, when the count of bases is within the preset range, the computer device 100 may input the nucleic acid sequence to the assumption model, and the assumption model may be additionally trained in such a manner that the host (or the organism) assumed using the input nucleic acid sequence is compared with label data (or label data for the organism) for the host of the training answer data.

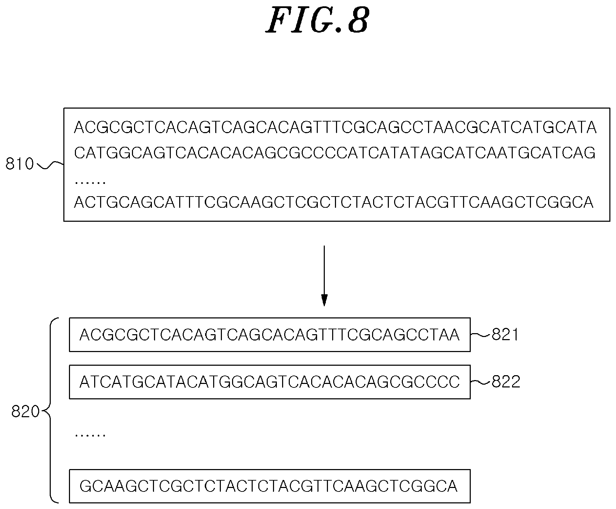

However, when the count of bases is out of the above-described preset range, the computer device 100 may perform pre-processing for dividing the nucleic acid sequence into partial sequences and may input the partial sequences to the assumption model. In this case, the assumption model may be additionally trained by comparing the host (or the organism) assumed using the input partial sequence and the label data for the host (or the label data for the organism) of the training answer data. This will be described with further reference to FIG. 8.

FIG. 8 exemplarily illustrates a process in which the nucleic acid sequence is preprocessed into partial sequences according to an embodiment.

As illustrated in FIG. 8, in the process of fine-tuning, when the count of bases included in the nucleic acid sequence 810, which is the training input data, exceeds the second cutoff the computer device 100 may obtain one or more partial sequences 820 from the nucleic acid sequence 810 in such a manner that the count of bases not greater than the second cutoff are included in each partial sequence.

In an embodiment, the computer device 100 may obtain a first partial sequence 821 including the count of bases not greater than the second cutoff from the nucleic acid sequence 810. For example, the computer device 100 may extract only one first partial sequence 821 including bases whose base count is the second cutoff (e.g., 20,000bp) when counted from a preset start point (e.g., a point of 300bp to 400 bp) from among the nucleic acid sequence 810 (e.g., 30,000bp). Also, the computer device 100 may provide only the first partial sequence 821 to the assumption model, and may not provide the remaining sequence excluding the first partial sequence 821 to the assumption model.

As described above, when the count of the partial sequence 820 is singular, the assumption model may assume the host (or the organism) for the partial sequence 820 and may be trained according to the result of comparing the assumed host (or the organism) with training answer data. For example, the assumption model may assume the host by using the first partial sequence 821, and may update weights of the model according to the result of comparing the assumed host with label data for the host of the nucleic acid sequence 810.

In another embodiment, the computer device 100 may obtain the plurality of partial sequences 820 each including the count of bases not greater than the second cutoff from the nucleic acid sequence 810. For example, the computer device 100 may divide the nucleic acid sequence 810 (e.g., 30,000bp) into the first partial sequence 821 including bases whose base count is the second cutoff (e.g., 20,000bp) when counted from the start point and the second partial sequence 822 including bases whose base count is the second cutoff (e.g., 20,000bp) when counted from the end point. Also, the computer device 100 may provide each of the divided first partial sequence 821 and the divided second partial sequence 822 to the assumption model.

In another embodiment, the computer device 100 may obtain the plurality of partial sequences 820 including the count of bases not less than the first cutoff and not greater than the second cutoff from the nucleic acid sequence 810. For example, the computer device 100 may divide the nucleic acid sequence 810 (e.g., 30,000bp cutoff) into the first partial sequence 821 including bases whose base count is the second cutoff (e.g., 20,000bp) when counted from the start point and the second partial sequence 822 including the remaining bases (e.g., 10,000bp) whose base count is not less than the first cutoff (e.g., 2,000bp) and not greater than the second cutoff (e.g., 20,000bp) when counted from the end point of the divided first partial sequence 821. As another example, the computer device 100 may divide the nucleic acid sequence 810 (e.g., 30,000bp) into the first partial sequence 821 and the second partial sequence 822 by dividing the nucleic acid sequence 810 in half. In addition, similarly, the computer device 100 may provide each of the divided first partial sequence 821 and the divided second partial sequence 822 to the assumption model.

As described above, when the number of the partial sequence 820 is plural, the assumption model according to an embodiment may assume a host (or an organism) for each of the plurality of partial sequences 820 and may statistically process the assumption results of the host (or the organism) for each of the plurality of partial sequences 820. In addition, the assumption model may determine the finally assumed host (or organism) by using the statistically processing result, and may be trained according to a comparison result between the host (or the organism) assumed for the nucleic acid sequence 810 and the training answer data. For example, the assumption model may be trained by a method of determining the host for the nucleic acid sequence 810, which is the entire sequence, according to the statistically processing result of a first host assumed using the first partial sequence 821, a second host assumed using the second partial sequence 822, to a Pth host assumed using a Pth partial sequence (P is a natural number not less than 3), and updating weights of the model according to the comparison result between the determined host and the label data for the host of the nucleic acid sequence 810.

The above-described statistical processing may include at least one of a Majority vote method, a mean method, and a standard deviation method. In an embodiment, the Majority vote method may include at least one of a simple majority vote method in which a host (or organism) occupying the largest number is selected among the assumed host (or organism) for each of the multiple partial sequences (820), and an absolute majority vote method in which a host (or an organism) occupying the majority is selected. In an embodiment, the mean method (or the standard deviation method) may be a method in which an average value per category (or a standard deviation value per category) is obtained by averaging probability values per category of host (or an organism) assumed for each of the plurality of partial sequences 820, and a host(s) (or an organism(s)) whose average value per category (or standard deviation value per category) is the largest or not less than a reference value is selected. Although the Majority vote method, the mean method, and the standard deviation method are illustrated above, the present disclosure is not limited thereto, and various statistical processing methods may be used.

When the number of the partial sequence 820 is plural, the assumption model according to another embodiment may be trained according to the comparison result between the host (or organism) assumed for each of the plurality of partial sequences 820 and the training answer data. For example, the assumption model may be further trained for each in such a manner that the weights of the models are updated based on the comparison result between the first host and the label data for the host of the nucleic acid sequence 810 and the weights of the models are updated based on the comparison result between the second host and the label data for the host of the nucleic acid sequence 810.

According to an embodiment, the above-described points (e.g., the start point, the end point, etc.) used as a reference position for dividing the nucleic acid sequence 810 into the one or more partial sequences 820 may be changed in the process of fine-tuning. As an example, the above-described points may be set to random values within a predetermined range each time. As another example, the start point or the end point described above may be managed as a kind of hyperparameter, and for example, a value of the start point or the end point showing a higher assumption accuracy from among a plurality of assumption model candidates implemented by fine-tuning the pre-trained model 510 pre-trained with different start point values or end point values may be applied to the assumption model.

As described above, when the length of the nucleic acid sequence is not less than a predetermined length, the computer device 100 may preprocess the sequences in such a manner that the sequences are divided into several partial sequences according to a predetermined rule or randomly, and then provide the preprocessed sequences to the assumption model. Accordingly, the length of the sequence input to the assumption model may be adjusted to be within a similar range, and assumption accuracy may be improved.

Meanwhile, according to an embodiment, at least one of the host information and the organism information may be used in the process of fine-tuning described above, and the assumption model for at least one of the host assumption and the organism assumption may be implemented as a result of the fine-tuning.

In a first embodiment, the assumption model may be fine-tuned to assume the host of the nucleic acid sequence by using host information. For example, the training answer data used in the process of fine-tuning may include label data for the biological category of host of the corresponding nucleic acid sequence. Also, as illustrated in FIG. 5, the assumption model in which the host assumption layer 520 is applied to the pre-trained model 510 may be trained by using these training data sets. In addition, the assumption model may be implemented as the training result, which is trained to assume a probability value per category of the host when the nucleic acid sequence is input.

In a second embodiment, the assumption model may be fine-tuned to assume the organism carrying the nucleic acid sequence by using organism information. For example, the training answer data used in the process of fine-tuning may include label data for the biological category of the organism carrying the corresponding nucleic acid sequence. In addition, by using these training data sets, in a similar manner to that illustrated in FIG. 5, the assumption model in which an organism assumption layer (see reference numeral 520) is applied to the pre-trained model 510 may be trained, and the assumption model may be implemented as the training result, which is trained to assume a probability value per category of the organism when the nucleic acid sequence is input.

In a third embodiment, the assumption model may be fine-tuned to assume the host and organism of the nucleic acid sequence by using the host information and the organism information. For example, the training answer data used in the process of fine-tuning may include label data for the biological category of the organism carrying the corresponding nucleic acid sequence and label data for the biological category of host of the organism. In addition, the assumption model in which an assumption layer (see reference numeral 520) is applied to the pre-trained model 510 may be trained by using these training data sets, and the assumption model may be implemented as the training result, which is trained to assume the probability value per category of the host and the probability value per category of the organism together when the nucleic acid sequence is input. In this case, since the host species and the organism species can be assumed together with only the nucleic acid sequence as an input, it is possible to more efficiently verify whether the information on the host and the organism obtained together during the collection process of the nucleic acid sequence is accurate.

In a fourth embodiment, the assumption model may be fine-tuned to assume the host or organism of the nucleic acid sequence using the host information and the organism information.

As an example, in the fine-tuning process, the biological category of the nucleic acid sequence and the organism may be used as training input data, and the biological category of host for the nucleic acid sequence may be used as training answer data. Accordingly, the assumption model may be trained to output a probability value per category of the host considering two inputs when the nucleic acid sequence and the biological category of the organism are input together, and may be trained to get the host species right by comparing the output with the training answer data. In this case, since the organism information as well as the nucleic acid sequence may be used as the input, the host may be more accurately assumed.

As another example, the assumption model may be fine-tuned to assume the host by using the host information, and may be provided according to a type of a plurality of preset organisms. For example, a first assumption model obtained by performing the fine-tuning on the first pre-trained model 510 trained with training data of organisms belonging to a virus family, a second assumption model obtained by performing the fine-tuning on the second pre-trained model 510 trained with training data of organisms belonging to a bacterial family, a third assumption model obtained by performing the fine-tuning on the third pre-trained model 510 trained with training data of organisms belonging to a fungal family, and the like may be implemented. Alternatively, a fourth assumption model obtained by performing the fine-tuning on the pre-trained model 510 trained with training data of a large amount of organisms in order to assume the host of organisms belonging to a virus family, a fifth assumption model obtained by performing the fine-tuning on the pre-trained model 510 in order to assume the host of organisms belonging to a bacterial family, a sixth assumption model obtained by performing the fine-tuning on the fine-tuning on the pre-trained model 510 in order to assume the host of organisms belonging to a fungal family may be implemented. As described above, by classifying pathogen sequences in several predetermined categories such as the virus family and the bacteria family to implement the assumption model optimally fine-tuned for each of the pathogen families, the host can be more accurately assumed in consideration of the characteristics of nucleic acid sequences for each pathogen family.

Meanwhile, as another embodiment different from the above-described embodiment, the assumption model may be provided regardless of the type of the organism. For example, one assumption model may be implemented by using nucleic acid sequences whose type of organism is not limited as the training data set in the processes of pre-training and fine-tuning. In this case, the one assumption model can assume the host or organism from the nucleic acid sequences of all kinds of organisms.

Meanwhile, a plurality of hyper parameters may be used in each of the above-described processes of pre-training and fine-tuning. The hyper parameter may be a variable varied by a user. The hyper-parameter may include, for example, a learning rate, a cost function, the count of learning cycle iterations, weight initialization (e.g., setting a range of weight values that is a weight initialization target), and the count of Hidden Unit (e.g., the count of hidden layers and the count of nodes of the hidden layer). In addition, the hyper parameter may further include the tokenization technique (e.g., k-mer, gene prediction) described above, a set value of k in the case of the k-mer technique, a gradient accumulation step during gradient descent learning, a batch size, a drop out, and the like.

Meanwhile, although embodiments in which the assumption model is implemented by using the BERT-based transfer learning method have been mainly described throughout the specification, the assumption model is not limited thereto, and may be implemented by using various other neural networks (e.g., CNN, RNN, etc.) as the neural network-based model.

As described above, as will be described below, the assumption model may be learned by the transfer learning to output the assumption result for the host of the organism carrying the nucleic acid sequence or the organism when the nucleic acid sequence is input. The computer device 100 may store and manage the assumption model and may provide the assumption model. For example, the computer device 100 may be implemented in such a manner that the assumption model trained by the transfer learning method is stored and managed by the server, and the server provides the assumption model to the user terminal as the user terminal requests the assumption model.

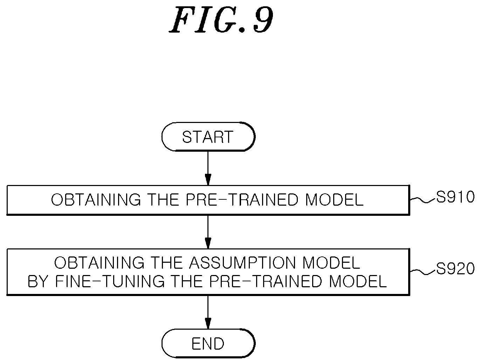

FIG. 9 illustrates an example flow diagram for obtaining the assumption model in accordance with an embodiment. FIG. 9 may be understood with reference to embodiments illustrated in FIGS. 1 to 8.

In an embodiment, the steps shown in FIG. 9 may be performed by the computer device 100. In an additional embodiment, the steps shown in FIG. 9 may be implemented by one entity such as the steps are performed by the server. In another embodiment, the steps shown in FIG. 9 may be implemented by a plurality of entities such as some of the steps are performed by the user terminal and the other steps are performed by the server.

In step S910, the computer device 100 may obtain the pre-trained model 510. In an embodiment, the computer device 100 may obtain the plurality of nucleic acid sequences 220, and may obtain the pre-trained model 510 by performing pre-training on the plurality of nucleic acid sequences 220. In another embodiment, the computer device 100 may receive the pre-trained model 510 pre-trained by another device from another device (or a storage unit) through the network.

In an embodiment, the pre-trained model 510 may use the plurality of nucleic acid sequences 220 as the training data. In an embodiment, the pre-trained model 510 may be trained by the semi-supervised learning method in such a manner that a mask to some of bases included in each of the plurality of nucleic acid sequences 220 is applied and then an answer to the masked base is made right. In an embodiment, the pre-trained model 510 may be trained by using a nucleic acid sequence tokenized with tokens each having two or more bases. In an embodiment, each of the tokens may include bases tokenized by (i) dividing the nucleic acid sequence by k unit or (ii) dividing the nucleic acid sequence by the function unit.

In step S920, the computer device 100 may obtain the assumption model by fine-tuning the pre-trained model 510. In an embodiment, the computer device 100 may obtain the plurality of training data sets and perform fine-tuning on the pre-trained model 510 by using the plurality of training data sets.

In an embodiment, the fine-tuning is performed by using the plurality of training data sets, and each training data set may include (i) the training input data including a nucleic acid sequence and (ii) the training answer data including a label data for an organism carrying the nucleic acid sequence or a host of the organism. In an embodiment, the fine-tuning may include (i) tokenizing the nucleic acid sequence included in the training input data to obtain a plurality of tokens, (ii) assuming the organism or the host of the nucleic acid sequence included in the training input data by using a context vector generated from the plurality of tokens, and (iii) training the pre-trained model 510 in such a manner that a difference between the assumed result and the training answer data is reduced.

In the first embodiment, the computer device 100 may perform the fine-tuning on the pre-trained model 510 by using each training data set including the nucleic acid sequence and the label data for host, and may obtain the assumption model learned to assume the host of a nucleic acid sequence when the nucleic acid sequence is input, as the learning result.

In the second embodiment, the computer device 100 may perform the fine-tuning on the pre-trained model 510 by using each training data set including the nucleic acid sequence and the label data for the organism, and may obtain the assumption model learned to assume the organism of a nucleic acid sequence when the nucleic acid sequence is input, as the learning result.

In the third embodiment, the computer device 100 may perform the fine-tuning on the pre-trained model 510 by using each training data set including the nucleic acid sequence, the label data for the host, and the label data for the organism, and may obtain the assumption model learned to assume the host and the organism of a nucleic acid sequence when the nucleic acid sequence is input, as the learning result.

In the fourth embodiment, the computer device 100 may obtain the plurality of pre-trained models 510 trained using training data classified for each of a plurality of preset organism types, and obtain the plurality of assumption models as a result of fine-tuning each of the plurality of pre-trained models 510 by using training data sets including a nucleic acid sequence and label data for host. Alternatively, the computer device 100 may retrieve one pre-trained model 510, may perform the fine-tuning by using the training data sets classified for each of the plurality of preset organism types, respectively, and may obtain the plurality of assumption models that are fine-tuned for each of the plurality of organism types as the learning result.

In the fifth embodiment, the computer device 100 may obtain the host assumption model according to the first embodiment and the organism assumption model according to the second embodiment, respectively, and obtain the assumption model including the host assumption model and the organism assumption model. According to embodiments, the host assumption model and the organism assumption model may have different model structures, or the types of neural networks for each model may be different.

In the above, various embodiments of the process in which the assumption model is learned in the transfer learning method have been described. As described above, according to an embodiment, in consideration that sequences of oligonucleotides (e.g., primers and probes) used in the molecular diagnostic reagent correspond to part of sequences of the target analyte (e.g., virus), technical features are proposed in which the assumption model is obtained by pre-training using nucleic acid sequences of the organism and fine-tuning for host assumption (or organism assumption). Accordingly, it is possible to implement the assumption model having high assumption performance even when a relatively small amount of labeled data is used. In addition, there is no need to prepare several assumption models according to the type of living organism, and there is an effect that allows for assuming the host or the organism from nucleic acid sequences of various types of organisms with only one assumption model.

Hereinafter, various embodiments of a process of assuming the host or the organism by using the assumption model presented above will be described.

The computer device 100 may receive the assumption model. In an embodiment, the computer device 100 may be implemented in such a manner that the assumption model is trained in the transfer learning method by the server and the user terminal receives the assumption model from the server.

The computer device 100 may obtain a nucleic acid sequence for assumption. Here, the nucleic acid sequence includes at least a portion of a nucleic acid sequence of which a host or an organism is desired to be known. In an embodiment, the computer device 100 may obtain the nucleic acid sequence from the public database or the memory 110 or through a user input. For example, the computer device 100 may receive a nucleic acid sequence of a living organism (e.g., Rotavirus A) registered in the public database such as NCBI or GISAID and may store the nucleic acid sequence in the memory 110.

In an embodiment, the computer device 100 may obtain sequence-related information including information about a nucleic acid sequence and the organism and/or the host of the nucleic acid sequence, and may obtain the nucleic acid sequence from the sequence-related information. For example, the sequence-related information including the nucleic acid sequence and the type of the organism and/or the type of the host may be collected from the public database, and in the following process, the type of the organism of the nucleic acid sequence and/or the host may be verified by using the assumption model.

The computing device 100 may provide the nucleic acid sequence to the assumption model, and then, may assume the organism carrying the nucleic acid sequence or the host of the organism from the assumption model. As described above, the organism carrying the nucleic acid sequence may refer to an organism corresponding to the corresponding nucleic acid sequence, for example, an organism at least partially including the corresponding nucleic acid sequence as a genetic material. The computer device 100 may input the nucleic acid sequence for assuming the organism or the host to the assumption model, and the assumption model may output a category of the organism carrying the corresponding nucleic acid sequence or the host among the plurality of biological categories 620.

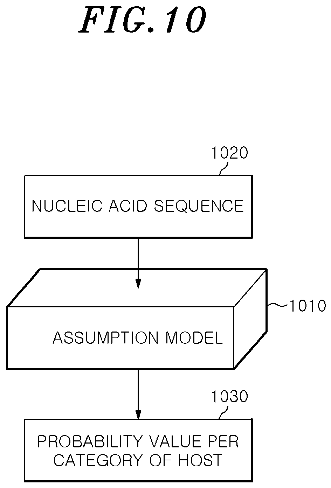

FIG. 10 is a conceptual diagram illustrating an inference operation of the assumption model 1010 according to an example embodiment. In an embodiment, the assumption model 1010 illustrated in FIG. 10 may refer to a model in which the fine-tuning or he transfer learning for the pre-trained model 510 is completed. Similarly, in FIG. 10, an inference process for host assumption according to an embodiment is illustrated, but in a similar manner, an inference process for organism assumption according to another embodiment may be performed.

Referring to FIG. 10, a nucleic acid sequence 1020 may be input to the assumption model 1010. As described above, the nucleic acid sequence 1020 is a nucleic acid sequence for which a host or an organism is to be known, and may mean, for example, sequence information in which bases located in at least a partial region of a genome of the organism are listed.

In an embodiment, in relation to the nucleic acid sequence 1020, the type of organism carrying the nucleic acid sequence may not be limited. For example, even if sequences of a specific organism species (e.g., Rotavirus A, Influenza virus A) were used in the process of fine-tuning, sequences of various organism species can be provided to the assumption model 1010 as inputs.

In another embodiment, in relation to the nucleic acid sequence 1020, the type of the organism carrying the nucleic acid sequence may be partially limited. For example, when sequences of the specific organism species (e.g., Rotavirus A, Influenza virus A) were used in the process of fine-tuning, nucleic acid sequences of organisms belonging to a preset biological category (e.g., virus neck) of the upper hierarchical level encompassing the corresponding species can be provided to the assumption model 1010 as inputs or a message recommending the same may be output.

In an embodiment, as the nucleic acid sequence 1020 is input, the assumption model 1010 may output a probability value 1030 per category of the host or a probability value per category of the organism. According to the above-described embodiments, the assumption model 1010 may output the probability value 1030 per category of host when it is learned to assume host, and may output the probability value per category of an organism when it is learned to assume an organism. Here, the probability value 1030 per category of host represents a probability value assumed that the host of the nucleic acid sequence 1020 corresponds to each of the plurality of predefined biological categories 620, and the probability value per category of the organism represents a probability value assumed that the organism of the nucleic acid sequence 1020 corresponds to each of the plurality of biological categories 620.

For example, as learned by fine-tuning in the above, the assumption model 1010 may output the probability value 1030 (e.g., 93%, 2%, 0.3%, etc.) per category of the host for each of the plurality of biological categories 620 (e.g., Homo sapiens, Sus scrofa, Bos taurus, etc.), which represents a probability value that the host of the nucleic acid sequence 1020 corresponds to each of the plurality of classes of the assumption model 1010.

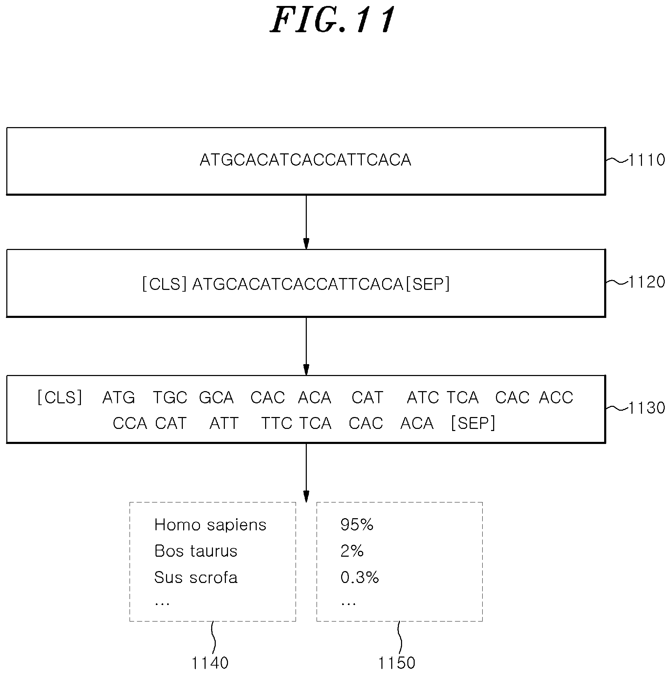

FIG. 11 illustrates an example scheme in which the assumption model 1010 assumes the probability value per category according to an embodiment.

Referring to FIG. 11, a nucleic acid sequence 1110 may be provided to the assumption model 1010 as input data. The nucleic acid sequence 1110 shown in FIG. 11 may correspond to the nucleic acid sequence 1020.

In an embodiment, the assumption model 1010 may obtain a sequence 1120 in which a special token is inserted into the nucleic acid sequence 1110. For example, the assumption model 1010 may insert a [CLS] token as a special token for indicating the start of the sequence at the first position of the nucleic acid sequence 1110, and may insert a [SEP] token as a special token for identifying the sequence at the last position.

In an embodiment, the assumption model 1010 may obtain the plurality of tokens 1130 corresponding to the sequence 1120 into which the special token is inserted. For example, at least one token may be generated with respect to each of bases included in the sequence 1020 into which the [CLS] token and the [SEP] token are inserted. As an example, the assumption model 1010 may receive the plurality of tokens 1130 as the nucleic acid sequence 1110. As another example, the assumption model 1010 may receive the nucleic acid sequence 1110 and perform preprocessing on the input nucleic acid sequence 1110 to generate the plurality of tokens 1130.

In an embodiment, each of the plurality of tokens 1130 may include a plurality of bases, and at least a portion of the nucleic acid sequence 1110 may overlap in tokens adjacent to each other among the plurality of tokens. According to example embodiments, the plurality of tokens 1130 may be obtained by using the k-mer technique or the gene assumption technique described above.

In an embodiment, the assumption model 1010 may output one or more assumption values 1150 corresponding to one or more classes 1140 of the assumption model 1010, by using the plurality of tokens 1130. According to an embodiment, each class 1140 may be any one of the plurality of predefined biological categories 620, and an assumption value 1150 corresponding to each class 1140 may be a probability value that a host or an organism corresponds to the corresponding biological category. For example, the computer device 100 may generate assumption values 1050 (e.g., the probability value 1030 per category of host) corresponding to the predetermined number of classes (e.g., the plurality of biological categories) for the plurality of tokens 1130 by using the assumption model 1010. In an embodiment, the type or number of classes of the assumption model 1010 may be variably determined according to an implementation aspect.

For example, as shown in FIG. 11, within a range in which the sum of the assumption values is 1, the first assumption value corresponding to the first class of the Homo sapiens may be 95%, the second assumption value corresponding to the second class of the Bos taurus may be 2%, and the third assumption value corresponding to the third class of the Sus scrofa may be 0.3%.

Meanwhile, the nucleic acid sequence 1020 provided to the assumption model 1010 may have the count of bases within a preset range. For example, the nucleic acid sequence 1020 input to the assumption model 1010 may have the count of bases either not less than the first cutoff or not greater than the second cutoff.

However, when the count of bases included in the nucleic acid sequence 1020 exceeds the preset range, the computer device 100 according to an embodiment may obtain one or more partial sequences from the nucleic acid sequence 1020 in such a manner that the count of bases is within the preset range, and provide the one or more partial sequences to the assumption model 1010.

Embodiments related thereto will be described with reference to FIG. 8 again. Some embodiments may include the above-described embodiments in the process of fine-tuning, and redundant descriptions will be omitted. In the following embodiments, the nucleic acid sequence 810 may be the nucleic acid sequence 1020 that is a target for assuming a host or an organism.

In an embodiment, when the count of bases exceeds the second cutoff, the computer device 100 may obtain one first partial sequence 821 from the nucleic acid sequence 810 in such a manner that bases whose base count is the second cutoff are included or bases whose base count is the second cutoff when counted from the preset start point are included. In addition, the computer device 100 may provide the first partial sequence 821 to the assumption model 1010, and may not provide the remaining sequences excluding the first partial sequence 821 to the assumption model 1010.

When the number of the partial sequence 820 is singular, the assumption model 1010 may assume the host of the organism carrying the first partial sequence 821 (or the organism) by using the input first partial sequence 821. The computer device 100 may obtain the assumed host for the first partial sequence 821 (or the assumed organism) from the assumption model 1010, and may output the assumed host as the host (or the organism) of the nucleic acid sequence 820.

In another embodiment, when the count of bases exceeds the second cutoff, the computer device 100 may obtain the plurality of partial sequences 820 from the nucleic acid sequence 810 in such a manner that the count of bases not greater than the second cutoff are included in each partial sequence. In addition, the computer device 100 may provide each of the plurality of partial sequences 820 to the assumption model 1010.

When the number of the partial sequences 820 is plural, the assumption model 1010 may assume the host (or the organism) for each of the plurality of partial sequences 820, and may output the assumption results of the host (or the organism) for each of the plurality of partial sequences 820. For example, the assumption model 1010 may output the probability value of host of the first partial sequence 821 by using the first partial sequence 821, and may output the probability value of host of the second partial sequence 822 by using the second partial sequence 822.

Alternatively, the computer device 100 may statistically process the assumption results of the host (or the organism) for each of the plurality of partial sequences 820, and may assume the host (or the organism) for the nucleic acid sequence 810 by using the statistically processing result. For example, the computer device 100 may select a category of host occupying the majority according to the majority vote method from among categories of host assumed for each of the plurality of partial sequences 820, or may select a category of host having the largest average of probability values for each category according to the average method, and may output the selected category of host as the host assumed finally for the nucleic acid sequence 820 which is the entire sequence.

In an embodiment, when outputting the assumed host (or organism) for the nucleic acid sequence 810, the computer device 100 may output the statistically processing result of the assumed host (or organism) for each of the plurality of partial sequences 820 together. For example, when the majority vote method is used, some other host types having a vote number not less than a reference value (e.g., 1) together with a highlight indication of the host type occupying the majority may be displayed. As another example, when the mean method is used, some other host types in which the average value for each category is not less than the reference value may be displayed, together with a highlight indication of the host type in which the average value for each category is the largest.

In an embodiment, when the count of bases is less than the first cutoff, the computer device 100 may output a message recommending input of a nucleic acid sequence including the count of bases not less than the first cutoff and not greater than the second cutoff.

Accordingly, the length of the nucleic acid sequence input to the assumption model 1010 may be adjusted to be within an appropriate range, and thus, when several assumption results are obtained, assumption accuracy can be improved by the statistical processing thereof.

In an embodiment, the computer device 100 may update the count of bases included in a nucleic acid sequence provided to the assumption model 1010 based on a assumption result of the assumption model 1010. Specifically, when the number of the assumed host (or organisms) is not less than a preset first count or not greater than a preset second count, the computer device 100 may update the first cutoff or the second cutoff to different values. For example, when the count of categories in which the probability value 1030 per category is not less than a preset reference value among the plurality of biological categories 620 output from the assumption model 1010 is greater than the first count (e.g., 3) or smaller than the second count (e.g., 1), the computer device 100 may update the first cutoff or the second cutoff to another preset value and input a nucleic acid sequence processed from the nucleic acid sequence to the assumption model 1010 in such a manner that the count of bases is not less than the updated first cutoff or not greater than the updated second cutoff, thereby re-assuming the host. Also, the computer device 100 may repeat the update and the re-assumption of the cutoff value until the re-assumption result satisfies a predetermined condition (e.g., the satisfaction of the count condition for the categories described above, the count of re-assumptions, or the like), and may provide the assumption result and/or the re-assumption result.

In an embodiment, when the count of organisms or host assumed by the assumption model 1010 is not less than the first count, the first cutoff may be updated to a larger value, and/or when the count of organisms or host assumed is not greater than the second count, the second cutoff may be updated to a smaller value. For example, as described above, when the count of categories having a probability value not less than a reference value among the plurality of biological categories 620 is greater than the first count, an inaccurate result is derived as a plurality of species are assumed, and thus, a nucleic acid sequence processed to include the increased count of bases may be input to the assumption model 1010 to obtain a stricter assumption result. Alternatively, when the count of categories whose probability value is not less than the reference value is less than the second count, an inaccurate result is derived as a suitable species is not assumed, and thus a nucleic acid sequence processed to include the decreased count of bases may be input to the assumption model 1010 to obtain a looser assumption result.

Typically, there are significant differences in the length of the genome or the length of the key sequence that identifies the organism depending on the type of organism. Accordingly, it is difficult to determine the length of an appropriate input sequence (the count of bases) used in a model, and thus, the accuracy of assumption results is reduced. However, according to the above-described embodiment, the length of the sequence input to the assumption model 1010 may be flexibly adjusted until an appropriate assumption result is obtained, and there is an advantage in that the above-described problem may be solved.

Meanwhile, the computer device 100 may output the host or the organism assumed by the assumption model 1010 in various ways.

In an embodiment, the computer device 100 may display a result screen including the nucleic acid sequence 1020 and the probability value 1030 per category of the host (or the probability value per category of the organism). For example, the organism name of each of the plurality of biological categories 620 and the probability value of each may be displayed on the result screen.

According to another embodiment, the computer device 100 may display a result screen including one or more hosts (or organisms) that satisfy a predetermined condition among the probability values 1030 per category (or probability values per category of the organisms) of the host. For example, on the result screen, the host species names which have the highest probability value or a probability value higher than the reference value among the probability values 1030 per category of host (or the probability values per category of the organism), or which are within the top specific count of host species names (or organism species names) when sorted in descending order, may be displayed together with the corresponding probability value.

The result screen may be displayed in the form of a table, or may be displayed in the form of a graph or an image according to an implementation aspect, and the type and scale of the table or the graph, and the like may be different.

In an embodiment, the computer device 100 may obtain and output the assumption basis data used in the assumption of the host (or the organism). For example, the computer device 100 may display a result screen including the assumed host and a basis request button together. In addition, when a user's selection input for the basis request button is received, the computer device 100 may obtain the assumption basis data for the probability value 1030 per category of host and display it. For example, a sequence fragment analyzed as a key feature in the assumption process of host among a series of nucleic acid sequences may be displayed as the assumption basis data. According to an embodiment, the assumption basis data may be displayed in the form of an image. For example, when the assumed host is Homo sapiens, a sequence fragment that serves as the key basis for assuming a uniquely high probability value only in humans from the nucleic acid sequence may be visually highlighted and displayed on the nucleic acid sequence image.

In an embodiment, the assumption basis data may be calculated in an explainable artificial intelligence (XAI) method. For example, the computer device 100 may provide descriptive information about the extracted feature or the like to a preset descriptive interface by using a method (e.g., SmoothGrad or the like) of extracting descriptive features from the assumption model 1010 implemented as the explainable deep learning model. According to an embodiment, in addition, a method (e.g., LRP, etc.) of checking features, weights, locations of key objects, and the like of input data depending on a model may be used. As another example, the computer device 100 may obtain the assumption basis data by using an attention algorithm used in the assumption model 1010. For example, the assumption basis data may be generated by using the similarity calculated according to the attention algorithm, the keys and values with the similarity applied, the attention values, and the like. In addition, in the process of obtaining the assumption basis data, a BERT internal structural analysis algorithm (see ACL 2019) or the like may be used.

Meanwhile, according to an embodiment, a model-independent description method (e.g., LIME) in which cause analysis is performed by checking the outputs derived by adjusting inputs without depending on the model, or the like may be used in the process of obtaining the assumption basis data.

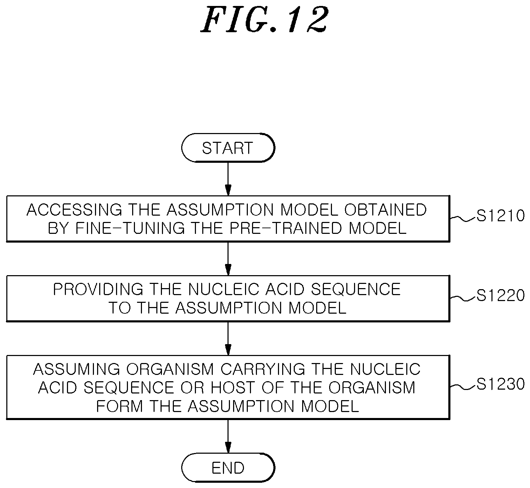

FIG. 12 illustrates an example flow diagram for assuming the organism or the host according to an embodiment. FIG. 12 may be understood with reference to the embodiments illustrated in FIGS. 1 to 11.

In an embodiment, the steps illustrated in FIG. 12 may be performed by the computer device 100. In an additional embodiment, the steps shown in FIG. 12 may be implemented by one entity, such as the steps are performed by the terminal. In another embodiment, the steps shown in FIG. 12 may be implemented by a plurality of entities, such as some of the steps shown in FIG. 12 are performed by the user terminal and the other steps are performed by the server.

In step S1210, the computer device 100 may access the assumption model 1010 obtained by fine-tuning the pre-trained model 510.

In an embodiment, the computer device 100 may be implemented to access the assumption model 1010 in such a manner that the user terminal receives the assumption model 1010 trained by the server in the transfer learning method from the server and executes the assumption model 1010. In another one embodiment, the computer device 100 may be implemented to access the assumption model 1010 in such a manner that the assumption model 1010 trained by the server is stored in the database and the user terminal accesses the database to receive and execute the assumption model 1010. In another one embodiment, the computer device 100 may be implemented to access the assumption model 1010 in such a manner that the user terminal loads and executes the assumption model 1010 previously stored in the memory 110 or another storage medium. In another one embodiment, the computer device 100 may be implemented to access the assumption model 1010 in such a manner that the user terminal transmits a request for execution of the assumption model 1010 trained by the server to the server together with data (e.g. input data) required to execute the assumption model 1010 and receives the execution result of the assumption model 1010 from the server. However, the access to the assumption model 1010 in the present disclosure is not limited thereto, and may be implemented in various modified forms.

In step S1220, the computer device 100 may provide the nucleic acid sequence 1020 to the assumption model 1010. In an embodiment, the computer device 100 may obtain the nucleic acid sequence 1020 based on a user input for the nucleic acid sequence, or may receive the nucleic acid sequence 1020 from the memory 110, another device, or storage medium. In an embodiment, the nucleic acid sequence 1020 to be assumed may be one of sequence candidates used in a diagnostic reagent for detection of a specific target analyte.

In an embodiment, the nucleic acid sequence 1020 provided to the assumption model 1010 may have the count of bases either not less than the preset first cutoff or not greater than the preset second cutoff.

In an embodiment, when the count of bases included in the nucleic acid sequence 1020 exceeds the second cutoff, the computer device 100 may obtain one or more partial sequences from the nucleic acid sequence 1020 in such a manner that the count of bases included not greater than the second cutoff are included. In this case, in step S1220, the one or more partial sequences may be provided to the assumption model 1010.

In an embodiment, in step S1220, one partial sequence including the count of bases not greater than the second cutoff when counted from the preset start point may be provided to the assumption model 1010, and the remaining sequence excluding the one partial sequence may not be provided to the assumption model 1010. Alternatively, in an embodiment, in step S1220, each of the plurality of partial sequences including the count of bases not greater than the second cutoff may be provided to the assumption model 1010.

In step S1230, the computer device 100 may assume an organism carrying the nucleic acid sequence 1020 or a host of the organism from the assumption model 1010.

In an embodiment, in the assumption, a category located in any one hierarchical level among the plurality of hierarchical levels constituting the biological classification system may be assumed, wherein the category is a biological category of the organism or the host. In an embodiment, the any one hierarchical level may be a species level in the biological classification system.

In an embodiment, when one partial sequence is provided to the assumption model 1010 in step S1220 and the remaining sequences are not provided to the assumption model 1010 in step S1230, a host (or an organism) for one partial sequence may be assumed, and the host (or the organism) assumed for the nucleic acid sequence 1020 may be the host for the one partial sequence.

In an embodiment, when each of the plurality of partial sequences is provided to the assumption model 1010 in step S1220, a host (or an organism) for each of the plurality of partial sequences may be assumed in step S1230. In this case, the computer device 100 may statistically process the assumption results of the host (or the organism) for each of the plurality of partial sequences, and the host (or the organism) assumed finally for the nucleic acid sequence 1020 may be obtained by using the statistically processing result of the host (or the organism) for each of the plurality of partial sequences.

The computer device 100 may provide different assumption results according to the above-described embodiments of fine-tuning.

In the first embodiment, the computer device 100 may provide the nucleic acid sequence 1020 to the assumption model 1010, and may provide an assumption result including the probability value 1030 per category of the host output from the assumption model 1010. For example, the assumption result may include the probability value (e.g., 95%, 3%, 0.1%, etc.) per category of the host for each of the plurality of biological categories (e.g., Homo sapiens, Bos taurus, Sus scrofa host, etc.) related to the host.

In the second embodiment, the computer device 100 may provide the nucleic acid sequence 1020 to the assumption model 1010, and may provide an assumption result including the probability value per category of the organism output from the assumption model 1010. For example, the assumption result may include the probability value (e.g., 95%, 3%, 0.1%, or the like) per category of the organism for each of the plurality of biological categories (e.g., Rotavirus A, Influenza virus A, Rabies lyssavirus, or the like) related to the organism.

In the third embodiment, the computer device 100 may provide the nucleic acid sequence 1020 to the assumption model 1010, and may provide an assumption result including the probability value 1030 per category of the host and the probability value per category of the organism output from the assumption model 1010.

In the fourth embodiment, the computer device 100 may obtain the plurality of assumption models 1010 prepared for each of the plurality of organism types, may obtain the nucleic acid sequence 1020 and the organism information from previously obtained sequence-related information, respectively, and may provide the nucleic acid sequence 1020 to any one assumption model 1010 corresponding to the corresponding organism information among the plurality of assumption models 1010. In addition, the computer device 100 may provide an assumption result including the probability value 1030 per category of host output from the assumption model 1010.

In the fifth embodiment, the computer device 100 may provide the nucleic acid sequence 1020 to each of the host assumption model and the organism assumption model included in the assumption model 1010, and provide an assumption result including the probability value 1030 per category of the host output from the host assumption model and the probability value per category of the organism output from the organism assumption model.

After step S1230, the computer device 100 according to an embodiment may obtain a comparison result between the information about the organism of the nucleic acid sequence 1020 and the assumed organism or a comparison result between the information about host and the assumed host.

Specifically, the computer device 100 may obtain the sequence-related information including the nucleic acid sequence 1020 and information about the organism and/or the host in step S1220 or before step S1220, may obtain the nucleic acid sequence 1020 from the sequence-related information, and may provide the obtained nucleic acid sequence 1020 to the assumption model 1020. For example, the computer device 100 may collect the sequence-related information from the public database. After step S1230, the computer device 100 may generate a comparison result between the biological category of the organism assumed by the assumption model 1020 and the biological category of the organism included in the sequence-related information, or may generate a comparison result between the biological category of the host assumed by the assumption model 1020 and the biological category of the host included in the sequence-related information. The comparison result may include, for example, whether the biological categories are identical and the probability value assumed for each biological category.

In an embodiment, the computer device 100 may output the comparison result or may transmit the comparison result to a user terminal used for development of a molecular diagnostic reagent in response to the user request.

As described above, by using the method for assuming the organism or the host described herein, the organism carrying the nucleic acid sequence or the host of the organism may be assumed from the nucleic acid sequence. This embodiment may not be combined with the technical features for obtaining the assumption model by the transfer learning method, and the technical features for assuming the organism or host using the assumption model may be independently used.

Hereinafter, various embodiments in which the assumption result of the organism or host presented above is used for the development of the molecular diagnostic reagent will be described. According to an embodiment, at least one of a primer and a probe used to detect a target analyte may be developed in the development of the molecular diagnostic reagent, but the present invention is not limited thereto.

In an embodiment, the nucleic acid sequence 1020 and the assumed host and/or the assumed organism may be used for a development of a molecular diagnostic reagent targeting the host or detection of the organism. For example, a development process of designing a sequence of a primer or probe used to detect whether a nucleic acid sequence of a specific pathogen is present in a sample collected from a specific target species of host (e.g., Homo sapiens) may be performed, and for this purpose, the sequence-related information on various nucleic acid sequences registered as sequences of the pathogen may be collected. For example, among the collected nucleic acid sequences, (i) a nucleic acid sequence verified that the host species assumed by the assumption model 1010 and the host species included in the sequence-related information are matched, and/or (ii) a nucleic acid sequence verified that the pathogen species assumed by the assumption model 1010 and the pathogen species included in the sequence-related information are matched may be used as one of candidate sequences for designing the primer or probe specific to the nucleic acid sequence of the corresponding pathogen in the development process.

In another embodiment, a comparison result between the information about the organism included in the sequence-related information and the assumed organism and/or a comparison result between the information about the host included in the sequence-related information and the assumed host may be used for a development of a molecular diagnostic reagent targeting the host and/or detecting the organism. For example, when the development process as described above is in progress, the sequence-related information including information about the organism to be detected in the development process, information about the host targeted in the development process, and a nucleic acid sequence corresponding thereto may be collected. Among the nucleic acid sequences included in the collected sequence-related information, only nucleic acid sequences verified that the host species assumed by the assumption model 1010 and the host species included in the sequence-related information are the same as each other, or the pathogen species assumed by the assumption model 1010 and the pathogen species included in the sequence-related information are the same as each other are used in the development process, and nucleic acid sequences confirmed to be different from each other may be excluded in the development process.

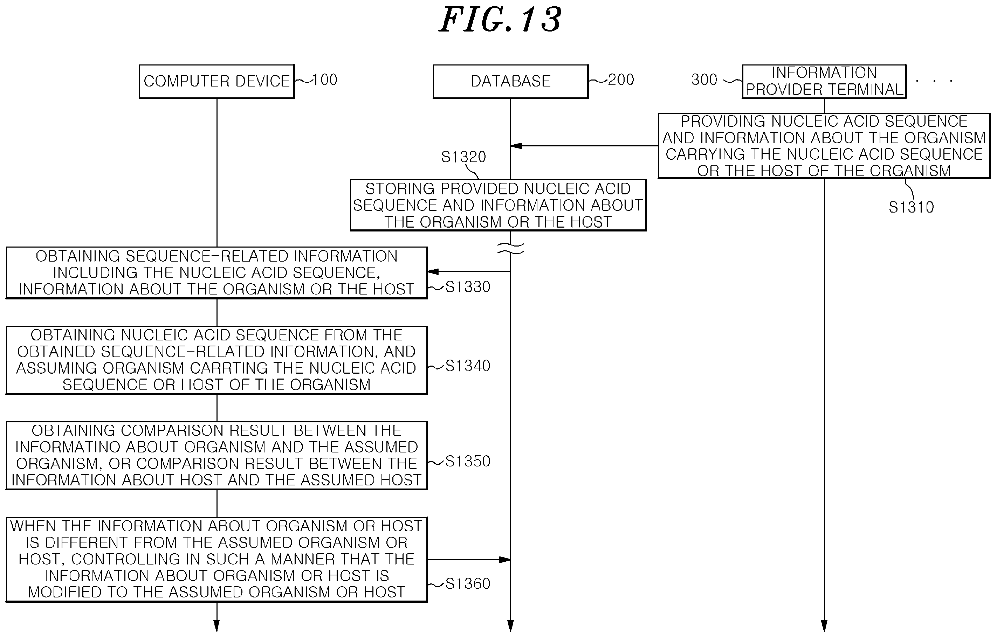

Embodiments related thereto will be described in more detail with reference to FIGS. 13 to 14.

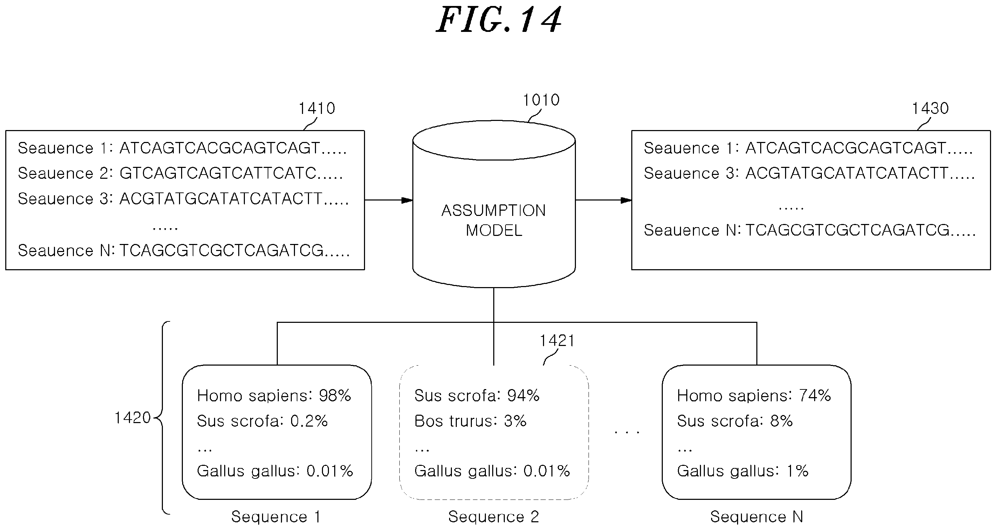

FIG. 13 is a flowchart illustrating a process in which the computer device 100 according to an embodiment allows the assumption result by an assumption model 1010 to be used for development of a molecular diagnostic reagent. In addition, FIG. 14 illustrates an exemplary method in which the computer device 100 controls the assumption result obtained by the assumption model 1010 to be used for the development of the molecular diagnostic reagent according to an embodiment.

Referring to FIGS. 13 to 14, the database 200 may be connected to the computer device 100 and one or more information provider terminals 300 through the network.

A plurality of nucleic acid sequences may be stored in the database 200. According to an embodiment, the database 200 may be implemented as a database accessible by a plurality of terminals, and may be, for example, the public database such as NCBI, or a private database provided by an operator of the computer device 100.

The information provider terminal 300 is a terminal of an information provider providing one or more nucleic acid sequences, and may include any type of terminal capable of interacting with a server or another computing device. Here, the information provider is a user capable of providing a nucleic acid sequence(s), and may be, for example, an institution, a company, a research institute, or the like, or an individual belonging thereto, but is not limited thereto. For example, the information provider may obtain information about a nucleic acid sequence of a specific pathogen included in a sample collected from the host by analysis of the sample or obtain information about a nucleic acid sequence of a specific pathogen and the corresponding host in other ways.

In step S1310, the information provider terminal 300 may provide the database 200 with the nucleic acid sequence and information about the organism carrying the nucleic acid sequence and/or the host of the organism. For example, the information provider terminal 300 may obtain the information about a nucleic acid sequence of a specific pathogen included in a sample collected from a host, a corresponding pathogen species, and a corresponding host species by analysis of the sample, and may register the obtained information to the database 200. As another example, the information provider terminal 300 may register a nucleic acid sequence of a specific pathogen, information about a corresponding pathogen species, and a species known as a corresponding host of the pathogen to the database 200.

In step S1320, the database 200 may store the provided nucleic acid sequence and the information about the organism carrying the nucleic acid sequence and/or the host of the organism. In an embodiment, the nucleic acid sequence received from the information provider terminal 300 may be classified by type of the organism or by type of the host and stored in the database 200.

According to an embodiment, the processes of steps S1310 to S1320 may be repeatedly performed by the plurality of information provider terminals 300, and accordingly, a plurality of nucleic acid sequences and information about the organisms and/or hosts may be collected in the database 200.

In step S1330, the computer device 100 may obtain the sequence-related information including the nucleic acid sequence, information about the corresponding organism, and/or the corresponding host from the database 200. In an embodiment, the computer device 100 may access the database 200, may search for information about nucleic acid sequences corresponding to the host and/or the organism targeted for reagent development from among various information about nucleic acid sequences provided by the database 200, and may obtain a plurality of sequence-related information including information about the corresponding host and the corresponding organism and the corresponding nucleic acid sequence as the search result corresponding thereto. Also, the computer device 100 may obtain a candidate nucleic acid sequence list 1410 including sequence-related information including a plurality of nucleic acid sequences corresponding to a specific target pathogen and a specific target host based on the obtained plurality of sequence-related information. For example, when the development of the molecular diagnostic reagent targeting the detection of an organism belonging to Rotavirus and a host belonging to Homo sapiens, the candidate nucleic acid sequence list 1410 may include nucleic acid sequences (e.g., Sequence 1 to N) included in sequence-related information in which information about the organism corresponds to Rotavirus and information about the host corresponds to Homosapiens, among the plurality of sequence-related information.

In step S1340, the computer device 100 may obtain the nucleic acid sequence from the obtained sequence-related information, and may assume the organism carrying the nucleic acid sequence or the host of the organism by using the assumption model 1010. In an embodiment, the computer device 100 may input each of the plurality of nucleic acid sequences included in the candidate nucleic acid sequence list 1410 to the assumption model 1010, and may obtain an assumption result 1420 of the host (or an assumption result of the organism) for each of the plurality of nucleic acid sequences from the assumption model 1010.

In step S1350, the computer device 100 may obtain the comparison result between the information about the organism included in the sequence-related information and the assumed organism, or the comparison result between the information about the host included in the sequence-related information and the assumed host. As described above, the comparison result may include whether the host (or the organism) included in the sequence-related information corresponds to the assumed host (or the assumed organism). In an embodiment, the comparison result may include at least one of whether the assumed probability value for the host (or the organism) included in the sequence-related information is the largest, whether the probability value is within the top preset count when sorted in descending order, and whether the probability value is not less than a reference value, among the probability values per category of the assumed host (or the organism). In an embodiment, the computer device 100 may output the comparison result. In another embodiment, the comparison result may be provided to the development process of the molecular diagnostic reagent targeting the corresponding host (or for detection of the corresponding organism).

In an embodiment, the computer device 100 may control a nucleic acid sequence that the host (or organism) included in the sequence-related information is different from the assumed host (or organism) not to be used for the development of the molecular diagnostic reagent targeting the host (or for detection of the organism). For example, the computer device 100 may detect an assumption result 1421 in which the biological category (e.g., Homo sapiens) of the host included in the sequence related information and the biological category (e.g., Sus scrofa) of the assumed host are different from each other, and may obtain a filtered candidate nucleic acid sequence list 1430 in which the nucleic acid sequence (e.g., sequence 2) corresponding to the assumption result 1421 is excluded from the candidate nucleic acid sequence list 1410. According to an embodiment, the filtered candidate nucleic acid sequence list 1430 may be used in a process of searching for a sequence specific to a species of a target analyte (e.g., gene region, intergenic region) because it is found with a high frequency in the species of the target analyte and is rarely found in other species, based on sequence alignment, or may be used in a process of determining a designable region for a primer and/or a probe based on the same.

In an embodiment, the computer device 100 may control that nucleic acid sequences in which the host (or the organism) included in the sequence-related information and the assumed host (or the organism) are checked to be identical to each other are used in the development of the molecular diagnostic reagent targeting the corresponding host (or for detection of the corresponding organism). For example, the computer device 100 may provide the filtered candidate nucleic acid sequence list 1430 to the user terminal associated with the development of the molecular diagnostic reagent, thereby supporting the verified nucleic acid sequences to be used in the development process.

According to the above-described embodiments, nucleic acid sequences in which the target host in the design process of the oligonucleotide and the assumed host are different from each other or nucleic acid sequences in which the target organism to be detected and the assumed organism are different from each other are excluded from the development of the diagnostic reagent, and thus the oligonucleotide designed based thereon may have more robust performance. For example, when the target analyte is detected by using the designed oligonucleotide, an oligonucleotide capable of more specifically binding to the target analyte or more specifically detecting the pathogen in a sample collected from host can be designed, and the possibility of false-positive can be reduced.

In step S1360, when the information about the organism or the host included in the sequence-related information is different from the assumed organism or the host, the computer device 100 may control in such a manner that the information about the organism or the host is modified to the assumed organism or the assumed host. According to an embodiment, when the information about the host (or the organism) included in the sequence-related information is different from the assumed host (or the assumed organism), the computer device 100 may update the information about the host (or the organism) included in the sequence-related information to the assumed host (or the organism). In another embodiment, the computer device 100 may transmit the comparison result to the database 200, thereby requesting or recommending the database 200 to modify the registered host (or organism) of the nucleic acid sequence into the assumed host (or organism).

According to an embodiment, when the information error in the database 200 is corrected, potential benefits of users using the nucleic acid sequences registered in the public database for various purposes may be improved.

As described above, the assumption result of the organism or the host may be used for the development of the molecular diagnostic reagent targeting the host or for detecting the organism, but is not limited to the above-described embodiments. According to embodiments, in addition to known host species, the assumption result may be used to predict potential host candidates that may later appear as hosts due to the variation, etc. Alternatively, according to an embodiment, the assumption result may be used to predict similarity between hosts for nucleic acid sequences of different organism species and to study or analyze zoonosis or the like based thereon.

Those skilled in the art will appreciate that the various illustrative logical blocks, modules, processors, means, circuits, and algorithm operations described in relationship to the exemplary embodiments disclosed herein may be implemented by electronic hardware (for convenience, called "software" herein), various forms of program or design code, or a combination thereof. In order to clearly describe compatibility of the hardware and the software, various illustrative components, blocks, modules, circuits, and operations are generally illustrated above in relation to the functions of the hardware and the software. Whether the function is implemented as hardware or software depends on design limits given to a specific application or an entire system. Those skilled in the art may perform the function described by various schemes for each specific application, but it shall not be construed that the determinations of the performance depart from the scope of the present disclosure.

Various exemplary embodiments presented herein may be implemented by a method, a device, or a manufactured article using a standard programming and/or engineering technology. A term "manufactured article" includes a computer program, a carrier, or a medium accessible from a predetermined computer-readable storage device. For example, the computer-readable storage medium includes a magnetic storage device (for example, a hard disk, a floppy disk, and a magnetic strip), an optical disk (for example, a CD and a DVD), a smart card, and a flash memory device (for example, an EEPROM, a card, a stick, and a key drive), but is not limited thereto. Further, various storage media presented herein include one or more devices and/or other machine-readable media for storing information.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

## Claims

1. A computer-implemented method performed by a computer device using a memory, a processor, and one or more programs stored in the memory and configured to be executed by the processor, the computer-implemented method comprising:
accessing an assumption model obtained by fine-tuning a pre-learned model;
providing a nucleic acid sequence to the assumption model; and
assuming an organism carrying the nucleic acid sequence or a host of the organism from the assumption model.

2. The computer-implemented method of claim 1, wherein the assumption comprises assuming a category located in any one hierarchical level among a plurality of hierarchical levels constituting a biological classification system, wherein the category is a biological category of the organism or the host.

3. The computer-implemented method of claim 2, wherein the any one hierarchical level is a species level in the biological classification system.

4. The computer implementation method of claim 1, wherein the pre-trained model uses a plurality of nucleic acid sequences as a training data.

5. The computer-implemented method of claim 4, wherein the pre-trained model is trained by a semi-supervised learning method performed in such a manner that a mask to some of bases in the nucleic acid sequences is applied and then an answer to the masked base is made right.

6. The computer-implemented method of claim 4, wherein the pre-trained model is trained by using nucleic acid sequences tokenized with tokens each having two or more bases.

7. The computer-implemented method of claim 6, wherein the tokens each includes bases tokenized by (i) dividing the nucleic acid sequence by k unit (k is a natural number) or (ii) dividing the nucleic acid sequence by a function unit.

8. The computer-implemented method of claim 1, wherein the fine-tuning is performed by using a plurality of training data sets, and each training data set includes (i) a training input data including a nucleic acid sequence and (ii) a training answer data including a label data as to an organism carrying the nucleic acid sequence or a host of the organism.

9. The computer-implemented method of claim 8, wherein the fine-tuning comprises (i) tokenizing the nucleic acid sequence included in the training input data to obtain a plurality of tokens, (ii) assuming the organism or the host of the nucleic acid sequence included in the training input data by using a context vector generated from the plurality of tokens, and (iii) training the pre-trained model in such a manner that a difference between the assumed result and the training answer data is reduced.

10. The computer-implemented method of claim 1, wherein the nucleic acid sequence provided to the assumption model has a count of bases either not less than a preset first cutoff or not greater than a preset second cutoff.

11. The computer-implemented method of claim 1, wherein the method further comprises, when the count of bases included in the nucleic acid sequence exceeds a preset second cutoff, obtaining one or more partial sequences from the nucleic acid sequence in such a manner that a count of bases not greater than the second cutoff are included, and
wherein the providing the nucleic acid sequence comprises providing the one or more partial sequences to the assumption model.

12. The computer-implemented method of claim 11, wherein the providing the nucleic acid sequence to the assumption model comprises:
providing to the assumption model one partial sequence including a count of bases not greater than the second cutoff when counted from a preset start point and not providing the remaining sequence excluding the one partial sequence to the assumption model; or
providing to the assumption model each of a plurality of partial sequences including a count of bases not greater than the second cutoff.

13. The computer-implemented method of claim 11, wherein the assumption comprises assuming an organism or a host for each of the plurality of partial sequences when the number of the partial sequence is plural,
the method further comprises statistically processing the assumption results of the organism or the host for each of the plurality of partial sequences, and
wherein the organism or the host assumed finally is obtained by using the statistically processing result.

14. The computer-implemented method of claim 13, wherein the statistical processing comprises at least one of a Majority vote method, a mean method, and a standard deviation method.

15. The computer-implemented method of claim 1, wherein (i) the assumed organism or the assumed host and (ii) the nucleic acid sequence are used for a development of a molecular diagnostic reagent targeting the host.

16. The computer-implemented method of claim 15, wherein the development of the molecular diagnostic reagent includes a development of at least one of a primer and a probe used for detection of the organism.

17. The computer-implemented method of claim 1, wherein the providing the nucleic acid sequence comprises obtaining the nucleic acid sequence from a sequence-related information including the nucleic acid sequence and an information about the organism or the host, and
wherein the method further comprises obtaining a comparison result between the information about the organism and the assumed organism or a comparison result between the information about the host and the assumed host.

18. The computer-implemented method of claim 1, wherein the providing the nucleic acid sequence comprises obtaining the nucleic acid sequence from a sequence-related information including the nucleic acid sequence and an information about the organism or the host, and
wherein the method further comprises controlling in such a manner that the information about the organism or the host is modified to the assumed organism or the assumed host when the information about the organism or the host is different from the assumed organism or the assumed host.

19. The computer-implemented method of claim 1, wherein the nucleic acid sequence provided to the assumption model has a count of bases not less than a preset first cutoff or not greater than a preset second cutoff, and
wherein the method further comprises updating the first cutoff or the second cutoff to a different value when a count of the assumed organisms or the assumed hosts is not less than a preset first count or not greater than a second count.

20. The computer-implemented method of claim 19, wherein the updating comprises:
updating the first cutoff to a larger value when the count of the assumed organisms or the assumed hosts is not less than the first count, and updating the second cutoff to a smaller value when the count of the assumed organisms or the assumed hosts is not greater than the second count.

21. The computer-implemented method of claim 1, further comprising:
controlling in such a manner that the nucleic acid sequence is not used for a development of a molecular diagnostic reagent targeting the organism or the host, when a count of the assumed organisms or the assumed host is not less than a preset reference count.

22. A computer program stored in a computer-readable recording medium, programmed to perform each step included in the method of claim 1.

23. A computer-readable recording medium, storing a computer program programmed to perform each step included in the method of claim 1.

24. A computer device, comprising:
a memory configured to store at least one instruction; and
the at least one instruction, when executed by the processor, cause the processor to:
access an assumption model obtained by fine-tuning a pre-learned model;
provide a nucleic acid sequence to the assumption model; and
assume an organism carrying the nucleic acid sequence or a host of the organism from the assumption model.

25. A computer-implemented method performed by a computer device using a memory, a processor, and one or more programs stored in the memory and configured to be executed by the processor, the computer-implemented method comprising:
accessing an assumption model obtained by fine-tuning a pre-learned model;
providing a nucleic acid sequence to the assumption model; and
assuming an organism carrying the nucleic acid sequence or a host of the organism from the assumption model,
wherein the pre-trained model uses a plurality of nucleic acid sequences as a training data, and
wherein the fine-tuning is performed by using a plurality of training data sets, and each training data set includes (i) a training input data including a nucleic acid sequence and (ii) a training answer data including a label data for an organism carrying the nucleic acid sequence or a host of the organism.

26. The computer-implemented method of claim 25, wherein the fine-tuning comprises (i) tokenizing the nucleic acid sequence included in the training input data to obtain a plurality of tokens, (ii) assuming the organism or the host of the nucleic acid sequence included in the training input data by using a context vector generated from the plurality of tokens, and (iii) training the pre-trained model in such a manner than a difference between the assumed result and the training answer data is reduced.

27. A computer-implemented method performed by a computer device using a memory, a processor, and one or more programs stored in the memory and configured to be executed by the processor, the computer-implemented method comprising:
obtaining a pre-trained model; and
by fine-tuning the pre-trained model, obtaining an assumption model learned to assume an organism carrying a nucleic acid sequence or a host of the organism when the nucleic acid sequence is provided,
wherein the fine-tuning is performed by using a plurality of training data sets, and each training data set includes (i) a training input data including a nucleic acid sequence and (ii) a training answer data including a label data for an organism carrying the nucleic acid sequence or a host of the organism.

28. The computer-implemented method of claim 27, wherein the label data is a label data for a category located in any one hierarchical level among a plurality of hierarchical levels constituting a biological classification system, wherein the category is a biological category of the organism or the host.

29. The computer implementation method of claim 28, wherein the pre-trained model uses a plurality of nucleic acid sequences as a training data.

30. The computer-implemented method of claim 29, wherein the pre-trained model is trained by a semi-supervised learning method performed in such a manner that a mask to some of bases in the nucleic acid sequences is applied and then an answer to the masked base is made right.

31. The computer-implemented method of claim 29, wherein the pre-trained model is trained by using a nucleic acid sequence tokenized with tokens each having two or more bases.

32. The computer-implemented method of claim 31, wherein the tokens each includes bases tokenized by (i) dividing the nucleic acid sequence by k unit (k is a natural number) or (ii) dividing the nucleic acid sequence by a function unit.

33. The computer-implemented method of claim 27, wherein the fine-tuning comprises (i) tokenizing the nucleic acid sequence included in the training input data to obtain a plurality of tokens, (ii) assuming the organism or the host of the nucleic acid sequence included in the training input data by using a context vector generated from the plurality of tokens, and (iii) training the pre-trained model in such a manner that a difference between the assumed result and the training answer data is reduced.

34. The computer-implemented method of claim 27, wherein the assumption model in the obtaining the assumption model is learned to:
assume an organism or a host of one or more partial sequences when the one or more partial sequences are provided, wherein the one or more partial sequences are obtained from the nucleic acid sequence in such a manner that include a count of bases not less than a preset second cutoff when a count of bases included in the nucleic acid sequence exceeds the second cutoff.

35. The computer-implemented method of claim 34, wherein the assumption model in the obtaining the assumption model is learned to:
assume an organism or a host for each of a plurality of partial sequences when the number of the partial sequences is plural, statistically process the assumption results of the organism or the host for each of the plurality of partial sequences, and finally assume the organism or the host of the nucleic acid sequence by using the statistical processing result.
